# EUROPEAN PATENT APPLICATION

(11) **EP 2 112 235 A1**
(43) Date of publication of application: **28.10.2009**
(21) Application number: 08155136.8
(22) Date of filing: 24.04.2008
(51) Int. Cl.: C12Q 1/68

(54) **Compositions and methods for microRNA expression profiling of nasopharyngeal carcinoma**

(71) Applicant: Max-Planck-Gesellschaft zur Förderung der Wissenschaften e.V., 80539 München (DE); Universitätsklinikum des Saarlandes, 66424 Homburg/Saar (DE)
(72) Inventor: Meister, Gunter, 82110 Germering (DE); Zhu, Jiayun, 81371 München (DE); Grässer, Friedrich A., 66421 Homburg/Saar (DE)
(74) Representative: Bühler, Dirk

(57) **Abstract**

The present invention relates compositions and methods for microRNA expression profiling of nasopharyngeal carcinoma cells. In particular, the invention relates to a diagnostic kit of molecular markers for identifying one or more mammalian target cells exhibiting or having a predisposition to develop a phenotype associated with a nasopharyngical carcinoma, the kit comprising a plurality of nucleic acid molecules, each nucleic acid molecule encoding a microRNA sequence, wherein one or more of the plurality of nucleic acid molecules are differentially expressed in the target cells and in one or more healthy control cells, and wherein the one or more differentially expressed nucleic acid molecules together represent a nucleic acid expression signature that is indicative for the presence of or the phenotype associated with a nasopharyngeal carcinoma. The invention further relates to methods using such nucleic acid expression signatures for identifying one or more mammalian target cells exhibiting or having a predisposition to develop a phenotype associated with a nasopharyngeal carcinoma as well as for preventing or treating such a phenotype. Finally, the invention is directed to a pharmaceutical composition for the prevention and/or treatment of nasopharyngeal carcinomas.

## Description

### FIELD OF THE INVENTION

The present invention relates to compositions and corresponding methods for microRNA expression profiling of nasopharyngeal carcinoma cells.

### BACKGROUND

Nasopharyngeal carcinoma (NPC) is a non-lymphomatous, squamous-cell carcinoma that occurs in the epithelial lining of the nasopharynx. This type of malignancy is a comparably uncommon disease worldwide except in Southeast Asia, northern Africa, and Alaska, where NPC is endemic, and representative incidence figures have been given as 10-40 per 100.000 (Yu, M.C. and Yuan, J.M. (2002) Semin. Cancer Biol. 12, 421-429). NPC differs significantly from other cancers of the head and neck in its occurrence, causes, clinical behavior, and treatment.

Undifferentiated NPC has a strong association with human Epstein-Barr virus (EBV), even though it is accepted that there are other environmental, dietary (e.g., the consumption of salt-cured fish high in nitrosamines), and genetic (e.g., the human leukocyte antigen system) factors implicated in its etiology. Diagnosing NPC in the early stages thus requires a high index of clinical acumen and, although most cross-sectional imaging investigations show the tumor precisely, conformation is dependent on additional serological and/or histological analysis.

Nevertheless, an elevation of serum EBV antibody levels is considered an outstanding and characterizing feature of the tumor. Accordingly, non-invasive NPC screening usually involves serological testing for the presence of IgA antibodies directed to various EBV antigens. Commercially available detection assays typically rely on the EBV capsid antigen (VCA) and/or the EBV early antigen (EA) (cf., e.g., Ji, M.F. (2007) Br. J. Cancer 96, 623-630; Tang, J.W. et al. (2007) J. Clin. Virol. 40, 284-288).

There are two widely used formats for EBV IgA testing: the immunofluorescence assay (IFA) and the enzyme-linked immunosorbent assay (ELISA). However, several limitations may interfere with the general applicability of these procedures, in particular with respect to routine testing required, e.g., for regular NPC screening of high-risk populations in Southeast Asia. The conventional IFA generally suffers from a lack of standardization, both in the production of the slides and their reading - the latter being affected by observer bias and level of experience. The ELISA assay is more easily standardized but also subject to observer bias in its interpretation. Furthermore, both assay formats require large amounts of immunological reagents as well as sophisticated instrumentation, thus making such assays expensive.

Aside from these methodological constraints, it is also of note that such serological assay are often hampered by the fact that they typically are based on the analysis of only a single molecular marker which might affect detection reliability and/or accuracy. In addition, a single marker normally does not enable detailed predictions concerning latency stages, tumor progression, and the like. Thus, there is still a need for the identification of alternative molecular markers and assay formats overcoming the above limitations.

One approach to address this issue might be based on small regulatory RNA molecules, in particular on microRNAs (miRNAs) which, constitute an evolutionary conserved class of endogenously expressed small non-coding RNAs of 20-25 nucleotides (nt) in size that can mediate the expression of target mRNAs and thus - since their discovery about ten years ago - have been implicated with critical functions in cellular development, differentiation, proliferation, and apoptosis.

MiRNAs are produced from primary transcripts that are processed to stem-loop structured precursors (pre-miRNAs) by the RNase III Drosha. After transport to the cytoplasm, another RNase III termed Dicer cleaves of the loop of the pre-miRNA hairpin to form a short double-stranded (ds) RNA, one strand of which is incorporated as mature miRNA into a miRNA-protein (miRNP). The miRNA guides the miRNPs to their target mRNAs where they exert their function (reviewed, e.g. in Bartel, D.P. (2004) Cell 23, 281-292; He, L. and Hannon, G.J. (2004) Nat. Rev. Genet. 5, 522-531). Depending on the degree of complementarity between the miRNA and its target, miRNAs can guide different regulatory processes. Target mRNAs that are highly complementary to miRNAs are specifically cleaved by mechanisms identical to RNA interference (RNAi) and the miRNAs function as short interfering RNAs (siRNAs). Target mRNAs with less complementarity to miRNAs are either directed to cellular degradation pathways or are translationally repressed without affecting the mRNA level. However, the mechanism of how miRNAs repress translation of their target mRNAs is still a matter of controversy.

Emerging data indicate that dysregulation of miRNA expression may *inter alia* be associated with the development and/or progression of certain types of cancer. Two miRNAs, *miR-15* and *miR-16-1*, were shown to map to a genetic locus that is deleted in chronic lymphatic leukemia (CLL) and it was found that in about 70% of the CLL patients, both miRNA genes are deleted or down-regulated. Furthermore, down-regulation of *miR-143* and *miR-145* was observed in colorectal neoplasia, whereas expression of the miRNA *let-7* is frequently reduced in lung cancers. In fact, it has been speculated based on cancer-associated alterations in miRNA expression, the observation that miRNAs are frequently located at genomic regions involved in cancers, and their gene regulatory function that miRNAs may act as both tumor suppressors and oncogenes (reviewed, e.g., in Esquela-Kerscher, A. and Slack, F.J (2006) Nat. Rev. Cancer 6, 259-269; Calin, G.A. and Croce, C.M. (2007) J. Clin. Invest. 117, 2059-2066; Blenkiron, C. and Miska, E.A. (2007) Hum. Mol. Genet. 16, R106-R113).

More systematic bead-based flow cytometric miRNA expression analyses revealed a global miRNAs down-regulation in tumors indicating that miRNA profiling of host cells might indeed be suitable for cancer diagnosis (cf., e.g., Lu J. et al. (2005) Nature 435, 834-838) and various miRNAs whose expression appears characteristic for a particular tumor have been identified (Calin, G.A. and Croce, C.M. (2007), *supra*). However, to date only few of these aberrantly expressed miRNAs have been directly linked with clinically relevant prognostic factors for tumor development and/or progression.

Soon after the discovery of mammalian miRNAs, parasites such as diverse viruses (e.g., Epstein-Barr virus, Karposi's sarcoma-associated herpes virus or human cytomegalovirus) have been found to encode and to express miRNAs as well (cf., e.g., international patent application WO 2005/097205; Cullen, B.R. (2006) Nat. Genet. 38, S25-S30). In case of Epstein-Barr virus, for example, the miRNAs are found in two clusters in the viral genome that are differentially expressed in different tissues with respect to the virus' latency stage (Cullen, B.R. (2006), *supra*). However, the physiological significance of this finding remains as elusive as the function of the vast majority of viral miRNAs at all. The potential role - if any - viral miRNAs might have with respect to cancer pathology is still to be unraveled

Thus, there still remains a need for reliable molecular diagnostic markers that enable the rapid, reliable and cost-saving identification and/or treatment of cells exhibiting or having a predisposition to develop a phenotype associated with a nasopharyngeal carcinoma. In addition, there is also a continuing need for corresponding methods both for the identification and for the treatment of target cells displaying such a cancerogenous phenotype.

Accordingly, it is an object of the invention to provide such compositions and methods.

### SUMMARY OF THE INVENTION

In a first aspect, the invention relates to a diagnostic kit of molecular markers for identifying one or more mammalian target cells exhibiting or having a predisposition to develop a phenotype associated with a nasopharyngeal carcinoma, the kit comprising a plurality of nucleic acid molecules, each nucleic acid molecule encoding a microRNA sequence, wherein one or more of the plurality of nucleic acid molecules are differentially expressed in the target cells and in one or more healthy control cells, and wherein the one or more differentially expressed nucleic acid molecules together represent a nucleic acid expression signature that is indicative for the presence of or the predisposition to develop a phenotype associated with a nasopharyngeal carcinoma.

In preferred embodiments, the nasopharyngeal carcinoma is associated with a viral infection, preferably a viral infection caused by Epstein-Barr virus.

In specific embodiments, the nucleic acid expression signature comprises at least five nucleic acid molecules, preferably at least ten nucleic acid molecules.

The nucleic acid expression signature is particularly preferred to comprise at least one nucleic acid molecule encoding a target cell-derived microRNA sequence and at least one nucleic acid molecule encoding a virus-derived microRNA sequence.

In other preferred embodiments, the nucleic acid expression signature comprises at least one nucleic acid molecule encoding a microRNA sequence whose expression is up-regulated in the one or more target cells compared to the one or more control cells and at least one nucleic acid molecule encoding a microRNA sequence whose expression is down-regulated in the one or more target cells compared to the one or more control cells.

Preferably, the nucleic acid expression signature comprises one or more human target cell-derived nucleic acid molecules encoding microRNA sequences selected from the group consisting of hsa-miR-23a, hsa-miR-23b, hsa-miR200c, hsa-miR-320, hsa-miR-17-5p, and hsa-miR-652.

It is also preferred that the nucleic acid expression signature comprises one or more Epstein-Barr virus-derived nucleic acid molecules encoding microRNA sequences selected from the group consisting of ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22.

Particularly preferably, the nucleic acid expression signature comprises nucleic acid molecules encoding hsa-miR-23a, hsa-miR-23b, hsa-miR200c, hsa-miR-320, hsa-miR-17-5p, hsa-miR-652, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22.

In further particularly preferred embodiments, the expression of the nucleic acid molecules encoding hsa-miR-23a, hsa-miR-23b, hsa-miR200c, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22 is up-regulated and the expression of the nucleic acid molecules hsa-miR-320, hsa-miR-17-5p, and hsa-miR-652 is down-regulated in the in the one or more target cells compared to the one or more control cells.

In a second aspect, the invention relates to a method for identifying one or more mammalian target cells exhibiting or having a predisposition to develop a phenotype associated with a nasopharyngeal carcinoma, the method comprising: determining in the one or more target cells the expression levels of a plurality of nucleic acid molecules, each nucleic acid molecule encoding a microRNA sequence; determining the expression levels of the plurality of nucleic acid molecules in one or more healthy control cells; and identifying from the plurality of nucleic acid molecules one or more nucleic acid molecules that are differentially expressed in the target and control cells by comparing the respective expression levels obtained, wherein the one or more differentially expressed nucleic acid molecules together represent a nucleic acid expression signature, as defined herein, that is indicative for the presence of or the predisposition to develop a phenotype associated with a nasopharyngeal carcinoma.

In preferred embodiments of the inventive method, the nasopharyngeal carcinoma is associated with a viral infection, preferably a viral infection caused by Epstein-Barr virus.

In a third aspect, the invention relates to a method for preventing or treating a phenotype associated with a nasopharyngeal carcinoma in one or more mammalian target cells, the method comprising: identifying in one or more target cells a nucleic acid expression signature by using a method as defined herein; and modifying in the one or more cells the expression of one or more nucleic acid molecules encoding a microRNA sequence that is/are comprised in the nucleic acid expression signature in such way that the expression of a nucleic acid molecule whose expression is up-regulated in the one or more target cells is down-regulated and the expression of a nucleic acid molecule whose expression is down-regulated in the one or more target cells is up-regulated.

In a forth aspect, the invention relates to a pharmaceutical composition for the prevention and/or treatment in one or more mammalian target cells having a phenotype associated with a nasopharyngeal carcinoma, the composition comprising one or more nucleic acid molecules, each nucleic acid molecule encoding a sequence that is at least partially complementary to a microRNA sequence encoded by a nucleic acid molecule whose expression is up-regulated in the one or more target cells, as defined herein, and/or that corresponds to a microRNA sequence encoded by a nucleic acid molecule whose expression is down-regulated in the one or more target cells, as defined herein.

In preferred embodiments of the pharmaceutical composition, the human nasopharyngeal carcinoma is associated with an infection caused by Epstein-Barr virus.

Finally, in a fifth aspect, the invention relates to the use of said pharmaceutical composition for the manufacture of a medicament for the prevention and/or treatment of a nasopharyngeal carcinoma, preferably a nasopharyngeal carcinoma associated with an infection caused by Epstein-Barr virus.

### DESCRIPTION OF THE DRAWINGS

**Figure 1****: Validation of novel miRNA candidates by Northern blot analysis.**

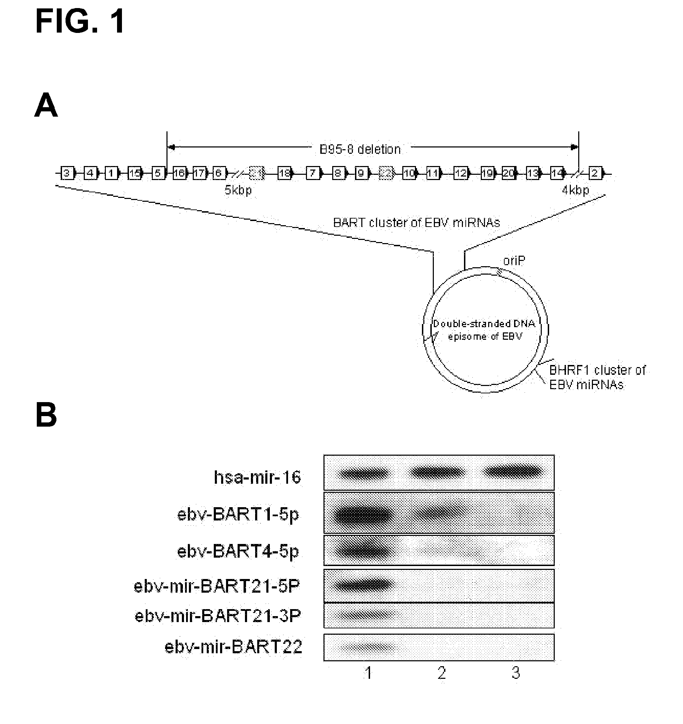

   Figure 1(A) depicts a schematic location of putative new miRNAs in BART miRNA cluster of EBV genome. Each box with an arrow indicates an EBV miRNA precursor and its transcription direction. The putative new miRNA precursors 21 and 22 are highlighted. The numbers in the box represent the name of the BART miRNAs. The genomic localization of the BHRF and BART clusters are schematically indicated in the double-stranded episome. OriP denotes the origin of replication. Figure 1(B) shows an expression analysis of novel EBV miRNAs by Northern blotting. Total RNA from Jijoye (lane 1), BL41/B95.8 (lane 2) and BL41 was transferred onto nylon membranes and hybridized with probes complementary to hsa-miR-16, ebv-miR-BART1-5p, ebv-miR-BART4, ebv-miR-BART21-5p, ebv-miR-BART21-3p and ebv-miR-BART22. The novel EBV miRNAs (i.e. ebv-miR-BART21-5p, ebv-miR-BART21-3p, and ebv-miR-BART22) are highlighted.
**Figure 2****: miRNA profiling in nasopharyngeal carcinoma (NPC) samples.**

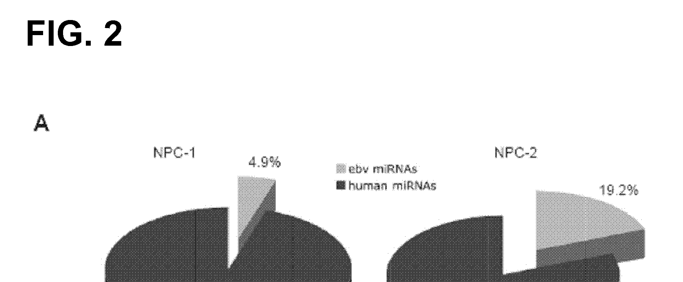

   Figure 2(A) depicts a schematic representation of cellular and EBV-derived miRNA fractions in NPC tissue samples. Figure 2(B) shows the relative abundance of EBV miRNAs in the NPC-1 and NPC-2 libraries (NPC-1 is shown in grey; NPC-2 is shown in black). Individual read numbers are presented as percentage of the total miRNA reads in the libraries. Figure 2(C) Cellular miRNAs hsa-miR-23a/b and hsa-miR-200c are up-regulated in NPC tissues, whereas hsa-miR-320, hsa-miR-17-5p, and hsa-miR-652 are more abundant in small RNA libraries form healthy tissue samples. Numbers of miRNA reads are shown as percentage of the total miRNA reads in the libraries.
**Figure 3****: hsa-mir-23 regulates tumor suppressor genes.**

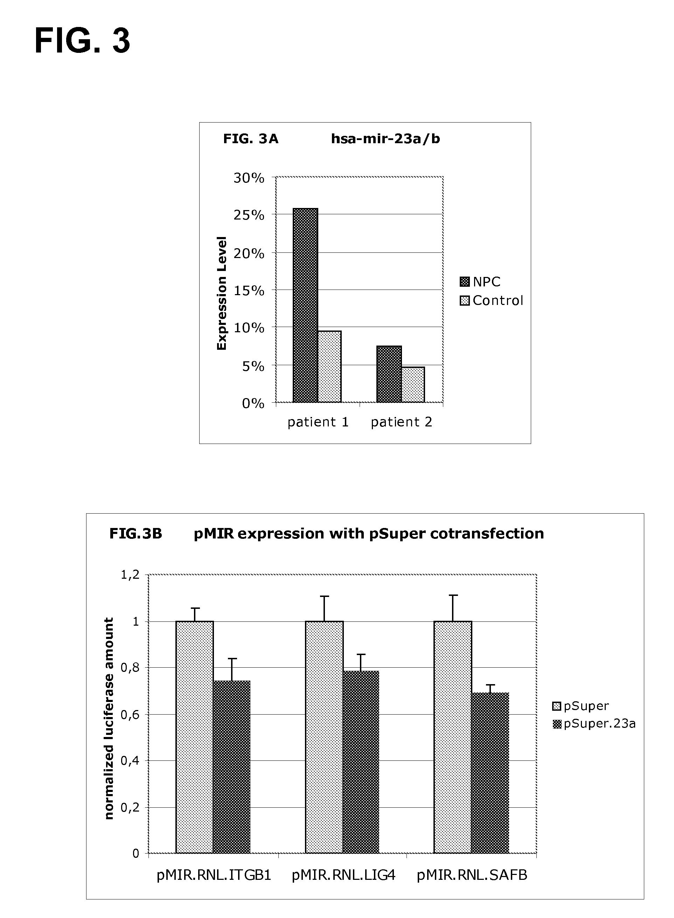

   Figure 3(A) illustrates that hsa-mir-23a and hsa-mir-23b are both up-regulated in NPC samples. Expression levels are shown as percentage of total microRNA reads in individual libraries. Figure 3(B) depicts that overexpression of hsa-mir-23a down-regulates a reporter construct consisting of the luciferase coding sequence fused to the 3'UTRs of different tumor suppressor genes harboring mir-23 regulatory elements. Thus, ITGB1, LIG4, SAFB appear to function as tumor suppressor genes in tumorigenesis and metastasis and are potential targets of mir-23. To this end, tumor suppressor gene 3'UTRs were cloned into individual pMIR-RNL luciferase reporter vectors (resulting in plasmids pMIR-RNL-ITGB1, pMIR-RNL-LIG4, and pMIR-RNL-SAFB). The stem-loop precursor sequence of hsa-mir-23a was cloned into pSUPER (resulting in plasmid pSuper.23a) for overexpression. After 48h cotransfection in 293T cells, luciferase was down-regulated in the presence of pSuper.23a compared to pSuper vector control.
**Figure 4****: hsa-mir-15 and hsa-mir-16 regulate tumor suppressor genes BRCA1.**

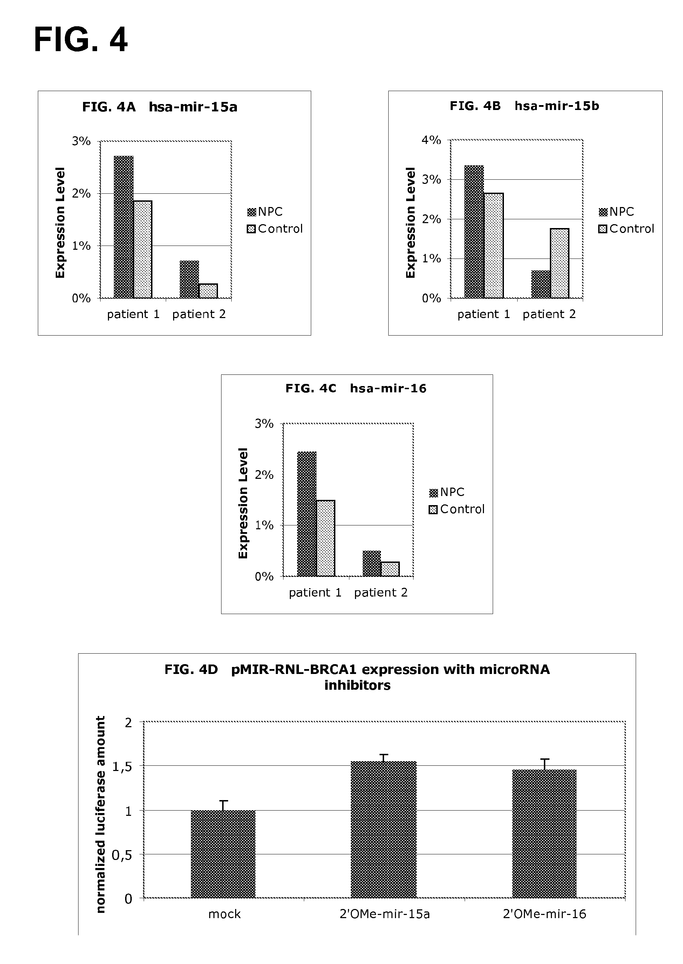

   Figures 4(A,B,C) illustrate that hsa-mir-15a, hsa-mir-15b and hsa-mir-16 are up-regulated in NPC samples. Expression levels are shown as percentage of total microRNA reads in individual libraries. Figure 4(D) demonstrates that inhibitors of hsa-mir-15a and hsa-mir-16 up-regulate a reporter construct consisting of the luciferase coding sequence fused to the 3'UTR of tumor suppressor gene BRCA1 harboring mir-15 and mir-16 regulatory elements. Thus, BRCA1 appears to function as a tumor suppressor gene in tumorigenesis and is a potential target of mir-15 and mir-16. To this end, the BRCA1 3'UTR was cloned into the pMIR-RNL luciferase reporter vector (resulting in plasmid pMIR-RNL-BRCA1). 2'O-methylation modified microRNA anti-sense sequences were used as microRNA inhibitors (2'O-Me-hsa-mir-15a, 2'O-Me-hsa-mir-16). After 48h cotransfection in Hela cells, luciferase was up-regulated in the presence of 2'OMe-hsa-mir-15a and 2'OMe-hsa-mir-16 compared to mock 2'OMe-microRNA.

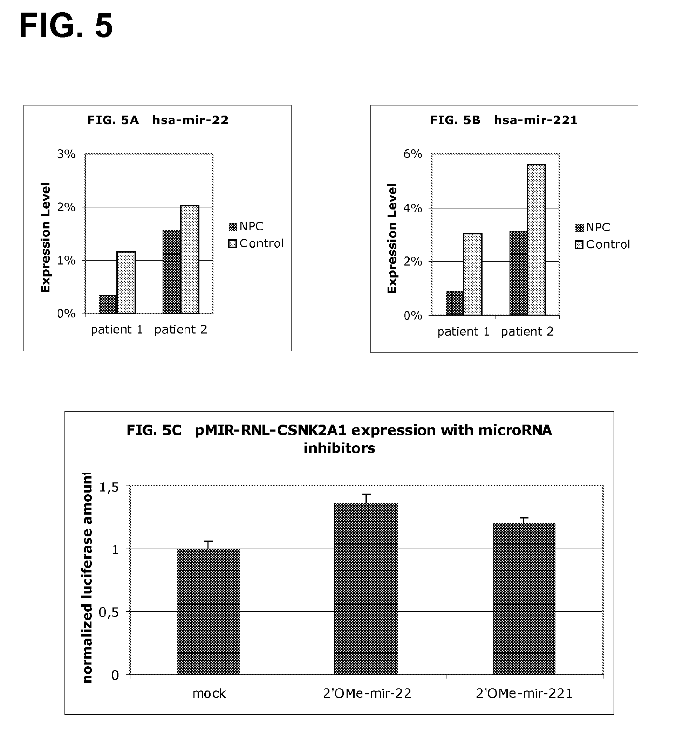

**Figure 5****: hsa-mir-22 and hsa-mir-221 regulate oncogene CSNK2A1.**
   Figure 5(A,B) illustrate that hsa-mir-22 and hsa-mir-221 are down-regulated in NPC samples. Expression levels are shown as percentage of total microRNA reads in individual libraries. Figure 5(C) demonstrates that inhibitors of hsa-mir-22 and hsa-mir-221 up-regulate a reporter construct consisting of the luciferase coding sequence fused to the 3'UTR of the oncogene CSNK2A1 harboring mir-22 and mir-221 regulatory elements. Thus, CSNK2A1 appears to function as an oncogene in tumorigenesis and is a potential target of hsa-mir-22 and hsa-mir-221. To this end, the CSNK2A1 3'UTR was cloned into the pMIR-RNL luciferase reporter vector (resulting in plasmid pMIR-RNL-CSNK2A1). 2'O-methylation modified microRNA anti-sense sequences are used and microRNA inhibitors (2'O-Me-hsa-mir-22, 2'O-Me-hsa-mir-221). After 48h cotransfection in Hela cells, luciferase was up-regulated in the presence of 2'OMe-hsa-mir-22 and 2'OMe-hsa-mir-221 compared to mock 2'OMe-microRNA.

### DETAILED DESCRIPTION OF THE INVENTION

The invention is based on the unexpected finding that cells exhibiting an EBV-positive human nasopharyngeal carcinoma can be reliably identified based on a miRNA expression signature comprising both differentially expressed human (target cell) miRNAs and EBV miRNAs, that is, viral infection appears in fact to be significantly implicated in cancer pathology. Furthermore, the present results imply a highly differentiated regulation. The expression signature comprises both up- and down-regulated human miRNAs, whereas the EBV miRNAs appear to be consistently up-regulated in the cancerogenous cells.

In a first aspect, the invention relates to a diagnostic kit of molecular markers for identifying one or more mammalian target cells exhibiting or having a predisposition to develop a phenotype associated with a nasopharyngeal carcinoma, the kit comprising a plurality of nucleic acid molecules, each nucleic acid molecule encoding a microRNA sequence, wherein one or more of the plurality of nucleic acid molecules are differentially expressed in the target cells and in one or more healthy control cells, and wherein the one or more differentially expressed nucleic acid molecules together represent a nucleic acid expression signature that is indicative for the presence of or the predisposition to develop a phenotype associated with a nasopharyngeal carcinoma.

The term "phenotype associated with a nasopharyngeal carcinoma" (herein also generally referred to as "cancerogenous phenotype"), as used herein, refers to any morphological and/or physiological alterations (that are, in turn, based on genetic re-programming) of target cells exhibiting or having a predisposition to develop characteristics of a nasopharyngeal carcinoma as compared to unaffected wild-type control cells. Examples of such alterations may relate *inter alia* to cell size and shape (enlargement or reduction), cell proliferation (increase in cell number), cell differentiation (change in physiological state), apoptosis (programmed cell death) or cell survival. The term "having a predisposition to develop a phenotype" denotes any cellular phenotype being indicative for a pre-cancerous state, i.e. an intermediate state in the transformation of a normal cell into a tumor cell.

The term "nasopharyngeal carcinoma (NPC)", as used herein, denotes a type of cancer originating in the nasopharynx, the uppermost region of the pharynx or "throat", where the nasal passages and auditory tubes join the remainder of the upper respiratory tract. NPC is classified as a malignant neoplasm, arising from the mucosal epithelium of the nasopharynx, most often within the lateral nasopharyngeal recess or fossa of Rosenmüller. There are three clinically relevant microscopic subtypes of NPC: a well-differentiated keratinizing type, a moderately-differentiated non-keratinizing type, and an undifferentiated type, which typically contains large numbers of non-cancerous lymphocytes (chronic inflammatory cells), thus giving rise to the name lymphoepithelioma. The undifferentiated form is most common, and is most strongly associated with Epstein-Barr virus infection.

The mammalian target cells employed in the present invention may be of human or non-human origin. However, the invention is typically performed with human cells. The term "one or more cells", as used herein, is to be understood not only to include individual cells but also tissues, organs, and organisms. The term "target cell", as used herein, refers to a cell being at least supposed to exhibit or to have a predisposition to develop phenotype associated with a nasopharyngeal carcinoma, whereas the term "control cell" denotes a wild-type cell not having characteristics of such a cancerogenous phenotype.

In a preferred embodiment of the invention, the phenotype associated with a nasopharyngeal carcinoma is associated with a viral infection. The term "associated with a viral infection" is to be understood that a virus has infected the cells exhibit or having a predisposition to develop such a phenotype. In other words, a virus can be detected in the target cells. The virus infecting the target cells may be any DNA or RNA virus (reviewed, e.g., in: Büchen-Osmond, C. (2003). Taxonomy and Classification of Viruses. In: Manual of Clinical Microbiology, 8th ed., vol. 2, p. 1217-1226, ASM Press, Washington DC). Examples of DNA viruses include *inter alia* the families of *Polyomaviridae* (e.g. SV 40 virus), *Adenoviridae* (e.g. adenovirus), and *Herpesviridae* (e.g. Epstein-Barr virus, Karposi's sarcoma-associated herpes, human cytomegalovirus). Examples of RNA viruses include *inter alia* the families of *Picornaviridae* (e.g. poliovirus, rhinovirus) *Flaviviridae* (e.g. hepatitis C virus), *Filoviridae* (e.g. Marburg virus), and *Retroviridae* (e.g. human immunodeficiency virus).

Within the scope of the invention, the phenotype associated with a nasopharyngeal carcinoma is typically associated with an infection of a single type of virus but it may also possible that the cells are infected with two or more different types of viruses. Preferably, the viruses infecting the target cells supposed to exhibit or to have a predisposition to develop such a cancerogenous phenotype encode in their genome one or more miRNAs.

Examples of such viruses are disclosed, e.g., in Cullen, B.R. (2006), *supra,* and include *inter alia* Karposi's sarcoma-associated herpes, human cytomegalovirus, and Epstein-Barr virus, with the latter one being particularly preferred.

The term "microRNA" (or "miRNA"), as used herein, is given its ordinary meaning in the art (reviewed, e.g. in Bartel, D.P. (2004) Cell 23, 281-292; He, L. and Hannon, G.J. (2004) Nat. Rev. Genet. 5, 522-531). Accordingly, a "microRNA" denotes a RNA molecule derived from a genomic locus that is processed from transcripts that can form local RNA precursor miRNA structures. The mature miRNA is usually 20, 21, 22, 23, 24, or 25 nucleotides in length, although other numbers of nucleotides may be present as well, for example 18, 19, 26 or 27 nucleotides.

The miRNA encoding sequence has the potential to pair with flanking genomic sequences, placing the mature miRNA within an imperfect RNA duplex (herein also referred to as stem-loop or hairpin structure or as pre-miRNA), which serves as an intermediate for miRNA processing from a longer precursor transcript. This processing typically occurs through the consecutive action of two specific endonucleases termed Drosha and Dicer. Drosha generates from the primary transcript (herein also denoted "pri-miRNA") a miRNA precursor (herein also denoted "pre-miRNA") that typically folds into a hairpin or stem-loop structure. From this miRNA precursor a miRNA duplex is excised by means of Dicer that comprises the mature miRNA at one arm of the hairpin or stem-loop structure and a similar-sized segment (commonly referred to miRNA*) at the other arm. The miRNA is then guided to its target mRNA to exert its function, whereas the miRNA* is degraded. In addition, miRNAs are typically derived from a segment of the genome that is distinct from predicted protein-coding regions.

The term "miRNA precursor" (or "precursor miRNA" or "pre-miRNA"), as used herein, refers to the portion of a miRNA primary transcript from which the mature miRNA is processed. Typically, the pre-miRNA folds into a stable hairpin (i.e. a duplex) or a stem-loop structure. The hairpin structures typically range from 50 to 80 nucleotides in length, preferably from 60 to 70 nucleotides (counting the miRNA residues, those pairing to the miRNA, and any intervening segment(s) but excluding more distal sequences).

The term "nucleic acid molecule encoding a microRNA sequence", as used herein, denotes any nucleic acid molecule coding for a microRNA (miRNA). Thus, the term does not only refer to mature miRNAs but also to the respective precursor miRNAs and primary miRNA transcripts as defined above. Furthermore, the present invention is not restricted to RNA molecules but also includes corresponding DNA molecules encoding a microRNA, e.g. DNA molecules generated by reverse transcribing a miRNA sequence. A nucleic acid molecule encoding a microRNA sequence according to the invention typically encodes a single miRNA sequence (i.e. an individual miRNA). However, it is also possible that such nucleic acid molecule encodes two or more miRNA sequences (i.e. two or more miRNAs), for example a transcriptional unit comprising two or more miRNA sequences under the control of common regulatory sequences such as a promoter or a transcriptional terminator.

A plurality of nucleic acid molecules as defined within the present invention may comprise at least two, at least 50, at least 100, at least 200, at least 500 or at least 10.000 nucleic acid molecules, each molecule encoding a miRNA sequence.

In specific embodiments, the plurality of nucleic acid molecules comprises at least one nucleic acid molecule encoding a target cell-derived mircoRNA sequence (e.g., a human miRNA) and at least one nucleic acid molecule encoding a virus-derived microRNA sequence (e.g., an EBV miRNA).

The term "differentially expressed", as used herein, denotes an altered expression level of a particular miRNA in the target cells as compared to the healthy control cells, which may be an up-regulation (i.e. an increased miRNA concentration in the target cells) or a down-regulation (i.e. a reduced or abolished miRNA concentration in the target cells). In other words, the nucleic acid molecule is activated to a higher or lower level in the target cells than in the control cells. Within the scope of the present invention, a nucleic acid molecule is to considered differentially expressed if the respective expression levels of this nucleic acid molecule in target cells and control cells typically differ by at least 5% or at least 10%, preferably by at least 20%, and most preferably by at least 30%. Thus, the latter value corresponds to an at least 1.3-fold up-regulation of the expression level of a given nucleic acid molecule in the target cells compared to the wild-type control cells or *vice versa* an at least 0.7-fold down-regulation of the expression level in the target cells.

The term "expression level", as used herein, refers to extent to which a particular miRNA sequence is transcribed from its genomic locus, that is, the concentration of a miRNA in the one or more cells to be analyzed.

The determining of expression levels typically follows established standard procedures well known in the art (cf., for example, Sambrook, J. et al. (1989) Molecular Cloning: A Laboratory Manual. 2nd Ed., Cold Spring Harbor Laboratory Press, Cold Spring Harbor, NY; Ausubel, F.M. et al. (2001) Current Protocols in Molecular Biology. Wiley & Sons, Hoboken, NJ). Determination may occur at the RNA level, for example by Northern blot analysis using miRNA-specific probes, or at the DNA level following reverse transcription (and cloning) of the RNA population, for example by quantitative PCR. The term "determining", as used herein, includes the analysis of any nucleic acid molecules encoding a microRNA sequence as described above. However, due to the short half-life of pri-miRNAs and pre-mRNAs typically the concentration of only the mature miRNA is measured.

Within the present invention, one or more differentially expressed nucleic acid molecules identified together represent a nucleic acid expression signature that is indicative for the presence of or the predisposition to develop a phenotype associated with a nasopharyngeal carcinoma in the target cells. The term "expression signature", as used herein, denotes a set of nucleic acid molecules (e.g., miRNAs), wherein the expression level of the individual nucleic acid molecules differs between the (cancerogenous) target cells and the (non-cancerogenous) control cells. Herein, a nucleic acid expression signature is also referred to as a set of markers and represents a minimum number of (different) nucleic acid molecules, each encoding a miRNA sequence that is capable for identifying a phenotypic state of a target cell.

In specific embodiments, the nucleic acid expression signature comprises at least five nucleic acid molecules, each encoding a (different) miRNA sequence, or at least 8 nucleic acid molecules. Preferably the nucleic acid signature comprises at least ten (different) nucleic acid molecules or at least twelve nucleic acid molecules.

In preferred embodiments, the nucleic acid expression signature comprises at least one nucleic acid molecule encoding a target cell-derived miRNA sequence (i.e. a miRNA sequence derived from the mammalian target cells, preferably human cells) and at least one nucleic acid molecule encoding a virus-derived microRNA sequence (i.e. a miRNA sequence derived from a virus infecting the target cells, such as an EBV miRNA). In a particularly preferred embodiment, the nucleic acid expression signature comprises at least three nucleic acid molecules encoding a target cell-derived miRNA sequence and at least three nucleic acid molecules encoding a virus-derived microRNA sequence.

In further preferred embodiments, the nucleic acid expression signature comprises at least one nucleic acid molecule encoding a miRNA sequence whose expression is up-regulated (i.e. its concentration is increased) in the one or more target cells compared to the one or more control cells and at least one nucleic acid molecule encoding a miRNA sequence whose expression is down-regulated (i.e. its concentration is reduced) in the one or more target cells compared to the one or more control cells.

In specific embodiments, the nucleic acid expression signature comprises at least two nucleic acid molecules, each encoding a target cell-derived miRNA sequence, wherein the expression of at least one of the at least two nucleic acid molecule is up-regulated and the expression of at least one other of the at least two nucleic acid molecule is down-regulated in the one or more target cells compared to the one or more control cells.

In other specific embodiments, the nucleic acid expression signature comprises at least one nucleic acid molecules encoding a virus-derived miRNA sequence whose expression is up-regulated in the one or more target cells compared to the one or more control cells. However, it may also be possible that the expression of nucleic acid molecules encoding a virus-derived miRNA sequence is down-regulated in the one or more target cells compared to the one or more control cells.

In preferred embodiments of the invention, the nucleic acid expression signature of the diagnostic kit comprises one or more human target cell-derived nucleic acid molecules encoding microRNA sequences selected from the group consisting of hsa-miR-23a (SEQ ID NO:1), hsa-miR-23b (SEQ ID NO:3), hsa-miR200c (SEQ ID NO:5), hsa-miR-320 (SEQ ID NO:7), hsa-miR-17-5p (SEQ ID NO:9), and hsa-miR-652 (SEQ ID NO:11). The respective miRNA precursor molecules for these mature miRNA sequences are given in SEQ ID NO:2, SEQ ID NO:4, SEQ ID NO:6, SEQ ID NO:8, SEQ ID NO:10, and SEQ ID NO:12.

All mature miRNA sequences as well as their corresponding precursors disclosed herein have been deposited in the miRBase database (http://microrna.sanger.ac.uk/; see also Griffiths-Jones S. et al. (2008) Nucl. Acids Res. 36, D154-D158).

In further preferred embodiments of the invention, the nucleic acid expression signature of the diagnostic kit comprises one or more Epstein-Barr virus-derived nucleic acid molecules encoding microRNA sequences selected from the group consisting of ebv-miR-BART1-5p (SEQ ID NO:13), ebv-miR-BART4 (SEQ ID NO:15), ebv-miR-BART6-3p (SEQ ID NO:17), ebv-miR-BART7-3p (SEQ ID NO:19), ebv-miR-BART11-3p (SEQ ID NO:21), ebv-miR-BART12 (SEQ ID NO:23), ebv-miR-BART19-3p (SEQ ID NO:25), and ebv-miR-BART22 (SEQ ID NO:27). The respective miRNA precursor molecules for these mature miRNA sequences are given in SEQ ID NO:14, SEQ ID NO:16, SEQ ID NO:18, SEQ ID NO:20, SEQ ID NO:22, SEQ ID NO:24, SEQ ID NO:26, and SEQ ID NO:28.

In specific embodiments, the nucleic acid expression signature of the diagnostic kit further comprises one or both of the Epstein-Barr virus-derived nucleic acid molecules selected from ebv-miR-BART21-3p (having the sequence 5'-CUAGUUGUGCCCACUGGUG UUU-3') and ebv-miR-BART21-5p (having the sequence 5'-UCACUAGUGAAGGCAA CUAAC-3').

In further specific embodiments, the nucleic acid expression signature is further **characterized in that** the Epstein-Barr virus-derived nucleic acid molecules comprised in the signature are all located in the *BART* miRNA cluster of the EBV genome (Pfeffer, S. et al. (2004) Science 304, 734-736). In other words, the nucleic acid expression signature according to the invention does not comprise miRNAs located in the *BHFR* miRNA cluster of the EBV genome. Particularly, the nucleic acid expression signature according to the present invention does not comprise ebv-miR-BHFR1 and/or ebv-miR-BHFR2 and/or ebv-miR-BHFR3. No expression of the latter three miRNAs was observed in NPC samples analyzed.

In particularly preferred embodiments, the nucleic acid expression signature comprises nucleic acid molecules encoding hsa-miR-23a, hsa-miR-23b, hsa-miR200c, hsa-miR-320, hsa-miR-17-5p, hsa-miR-652, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22.

In further particularly preferred embodiments, the expression of the nucleic acid molecules encoding hsa-miR-23a, hsa-miR-23b, hsa-miR200c, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22 is up-regulated and the expression of the nucleic acid molecules hsa-miR-320, hsa-miR-17-5p, and hsa-miR-652 is down-regulated in the in the one or more target cells compared to the one or more control cells.

In a second aspect, the invention relates to a method for identifying one or more mammalian target cells exhibiting or having a predisposition to develop a phenotype associated with a nasopharyngeal carcinoma, the method comprising:
(a) determining in the one or more target cells the expression levels of a plurality of nucleic acid molecules, each nucleic acid molecule encoding a microRNA sequence;
(b) determining the expression levels of the plurality of nucleic acid molecules in one or more healthy control cells; and
(c) identifying from the plurality of nucleic acid molecules one or more nucleic acid molecules that are differentially expressed in the target and control cells by comparing the respective expression levels obtained in steps (a) and (b),
wherein the one or more differentially expressed nucleic acid molecules together represent a nucleic acid expression signature, as defined in the present invention, that is indicative for the presence of or the predisposition to develop a phenotype associated with a nasopharyngeal carcinoma.

In preferred embodiments of the method, the nasopharyngeal carcinoma is associated with a viral infection, preferably a viral infection caused by Epstein-Barr virus.

The method of the present invention comprises determining and comparing the expression levels of a plurality of nucleic acid molecules encoding a microRNA sequence both in one or more target cells supposed to exhibit or to have the predisposition to develop a phenotype associated with a nasopharyngeal carcinoma and in one or more control cells, i.e. wild-type cells not showing the characteristics of such a cancerogenous cell (cf. the discussion above).

In a third aspect, the invention relates to a method for preventing or treating a phenotype associated with a nasopharyngeal carcinoma in one or more mammalian target cells, the method comprising:
(a) identifying in one or more target cells a nucleic acid expression signature by using a method as defined herein; and
(b) modifying in the one or more cells the expression of one or more nucleic acid molecules encoding a microRNA sequence that is/are comprised in the nucleic acid expression signature in such way that the expression of a nucleic acid molecule whose expression is up-regulated in the one or more target cells is down-regulated and the expression of a nucleic acid molecule whose expression is down-regulated in the one or more target cells is up-regulated.

The term "modifying the expression of a nucleic acid molecule encoding a miRNA sequence", as used herein, denotes any manipulation of a particular nucleic acid molecule resulting in an altered expression level of said molecule, that is, the production of a different amount of corresponding miRNA as compared to the expression of the wild-type. The term "different amount", as used herein, includes both a higher amount and a lower amount than wild-type. In other words, a manipulation, as defined herein, may either up-regulate (i.e. activate) or down-regulate (i.e. inhibit) expression (i.e. particularly transcription) of a nucleic acid molecule.

Within the present invention, expression of one or more nucleic acid molecules encoding a microRNA sequence comprised in the nucleic acid expression signature is modified in such way that the expression of a nucleic acid molecule whose expression is up-regulated in the one or more target cells is down-regulated and the expression of a nucleic acid molecule whose expression is down-regulated in the one or more target cells is up-regulated. In other words, the modification of expression of a particular nucleic acid molecule encoding a miRNA sequence occurs in an anti-cyclical pattern to the regulation of said molecule in the one or more cancerogenous target cells in order to interfere with the "excess activity" of an up-regulated molecule and/or to restore the "deficient activity" of a down-regulated molecule in the one or more target cells.

In a preferred embodiment of the inventive method, down-regulating the expression of a nucleic acid molecule comprises introducing into the one or more target cells a nucleic acid molecule encoding a sequence that is complementary to the microRNA sequence encoded by nucleic acid molecule to be down-regulated.

The term "introducing into a cell", as used herein, refers to any manipulation allowing the transfer of one or more nucleic acid molecules into a cell. Examples of such techniques include inter alia transfection or transduction techniques all of them well established in the art (cf., for example, Sambrook, J. et al. (1989) Molecular, Cloning: A Laboratory Manual, 2nd ed., Cold Spring Harbor Laboratory Press, Cold Spring Harbor, NY; Ausubel, F.M. et al. (2001) Current Protocols in Molecular Biology, Wiley & Sons, Hoboken, NJ).

The term "complementary sequence", as used herein, is to be understood that the "complementary" nucleic acid molecule (herein also referred to as an "anti-sense nucleic acid molecule") introduced into the one or more cells is capable of forming base pairs, preferably Watson-Crick base pairs, with the up-regulated endogenous "sense" nucleic acid molecule. The two nucleic acid molecules (i.e. the "sense" and the "anti-sense" molecule) may be perfectly complementary, that is, they do not contain any base mismatches and/or additional or missing nucleotides. In other embodiments, the two molecules comprise one or more base mismatched or differ in their total amounts of nucleic acid molecules). In further embodiments, the "complementary" nucleic acid molecule comprises at least ten contiguous nucleotides showing perfect complementarity with a sequence comprised in the up-regulated "sense" nucleic acid molecule.

The "complementary" nucleic acid molecule (i.e. the nucleic acid molecule encoding a nucleic acid sequence that is complementary to the microRNA sequence encoded by nucleic acid molecule to be down-regulated) may be a naturally occurring DNA- or RNA molecule or a synthetic nucleic acid molecule comprising in its sequence one or more modified nucleotides which may be of the same type or of one or more different types.

For example it may be possible that such a nucleic acid molecule comprises at least one ribonucleotide backbone unit and at least one deoxyribonucleotide backbone units. Furthermore, the nucleic acid molecule may contain one or more modifications of the RNA backbone into 2'-*O*-methyl group or 2'-*O*-methoxyethyl group (also referred to as "2'-*O-*methylation"), which prevented nuclease degradation in the culture media and, importantly, also prevented endonucleolytic cleavage by the RNA-induced silencing complex nuclease, leading to irreversible inhibition of the miRNA. Another possible modification - which is functionally equivalent to 2'-*O*-methylation - involves locked nucleic acids (LNAs) representing nucleic acid analogs containing one or more LNA nucleotide monomers with a bicyclic furanose unit locked in an RNA-mimicking sugar conformation (cf., e.g., Orom, U.A. et al. (2006) Gene 372, 137-141). Another class of silencers of miRNA expression were recently developed. These chemically engineered oligonucleotides, named "antagomirs", represent single-stranded 23-nucleotide RNA molecules conjugated to cholesterol (Krutzfeldt, J. et al. (2005) Nature 438, 685-689). As an alternative to such chemically modified oligonucleotides, microRNA inhibitors that can be expressed in cells, as RNAs produced from transgenes, were generated as well. Termed "microRNA sponges", these competitive inhibitors are transcripts expressed from strong promoters, containing multiple, tandem binding sites to a microRNA of interest (Ebert, M.S. et al. (2007) Nat. Methods 4, 721-726).

In particularly preferred embodiments of the inventive method, the one or more nucleic acid molecules whose expression is to be down-regulated encode microRNA sequences selected from the group consisting of hsa-miR-23a, hsa-miR-23b, hsa-miR200c, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22.

In a further preferred embodiment of the inventive method, up-regulating the expression of a nucleic acid molecule comprises introducing into the one or more target cells a nucleic acid molecule encoding the microRNA sequence encoded by nucleic acid molecule to be up-regulated. In other words, the up-regulation of the expression of a nucleic acid molecule encoding a miRNA sequence is accomplished by introducing into the one or more cells another copy of said miRNA sequence (i.e. an additional "sense" nucleic acid molecule). The "sense" nucleic acid molecule to be introduced into the one or more target cells may comprise the same modification as the "anti-sense" nucleic acid molecules described above.

In a particularly preferred embodiment, the one or more nucleic acid molecules whose expression is to be up-regulated encode microRNA sequences selected from the group consisting of hsa-miR-320, hsa-miR-17-5p, and hsa-miR-652.

The "sense" and/or the "anti-sense" nucleic acid molecules to be introduced into the one ore more target cells in order to modify the expression of one or more nucleic acid molecules encoding a microRNA sequence that is/are comprised in the nucleic acid expression signature may be operably linked to a regulatory sequence in order to allow expression of the nucleotide sequence.

In order to unravel any potential implication of the miRNAs identified in the NPC samples preliminary functional analyses were performed with respect to the identification of mRNA target sequences to which the miRNAs may bind. Based on the finding that miRNAs may be involved in both tumor suppression and tumorigenesis (reviewed, e.g., in Esquela-Kerscher, A. and Slack, F.J (2006) *supra*; Calin, G.A. and Croce, C.M. (2007) *supra*; Blenkiron, C. and Miska, E.A. (2007) *supra*) it is likely to speculate that mRNA target sites for such miRNAs include tumor suppressor genes as well as oncogenes.

And indeed, in exemplary analyses it was shown that human miRNAs whose expression is up-regulated in NPC samples regulate expression of tumor suppressor genes. In particular, hsa-mir-23a and hsa-mir-23b were experimentally demonstrated to regulate ITGB1 (integrin, beta-1), LIG4 (ligase-4), and SAFB (scaffold attachment factor B) tumor suppressor gene expression (cf. Figure 3). In addition, hsa-mir-15a, hsa-mir-15b and hsa-mir-16 were shown to regulate expression of the BRCA1 tumor suppressor gene (breast cancer 1, early onset, susceptibility gene) (cf. Figure 4). Structural prediction analysis further identified potential target sites for hsa-miR-15a, hsa-miR-16, and hsa-miR-497 within the CHK1 tumor suppressor gene (a serine/threonine protein kinase).

Vice versa, it was also shown that human miRNAs whose expression is down-regulated in NPC samples interact with oncogenes and affect their expression. Experimental evidence is available that hsa-mir-22 and hsa-mir-221 regulate expression of the oncogene CSNK2A1 (casein kinase 2 alpha-1) (cf. Figure 5). In addition, structural prediction analyses revealed potential target sites for hsa-miR-134, hsa-miR-190, and hsa-miR-196a within the c-MYC oncogene and for hsa-miR-320 within the β-catenin gene, respectively.

A nucleic acid molecule is referred to as "capable of expressing a nucleic acid molecule" or capable "to allow expression of a nucleotide sequence" if it comprises sequence elements which contain information regarding to transcriptional and/or translational regulation, and such sequences are "operably linked" to the nucleotide sequence encoding the polypeptide. An operable linkage is a linkage in which the regulatory sequence elements and the sequence to be expressed (and/or the sequences to be expressed among each other) are connected in a way that enables gene expression. The precise nature of the regulatory regions necessary for gene expression may vary among species, but in general these regions comprise a promoter which, in prokaryotes, contains both the promoter *per se*, i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'-capping elements in eukaryotes. These regions can also include enhancer or repressor elements as well as translated signal and leader sequences for targeting the native polypeptide to a specific compartment of a host cell.

In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

Furthermore, the expression of the nucleic molecules, as defined herein, may also be influenced by the presence, e.g., of modified nucleotides (cf. the discussion above). For example, locked nucleic acid (LNA) monomers are thought to increase the functional half-life of miRNAs *in vivo* by enhancing the resistance to degradation and by stabilizing the miRNA-target duplex structure that is crucial for silencing activity (cf., e.g., Naguibneva, I. et al. (2006) Biomed. Pharmacother. 60, 633-638).

Therefore, a nucleic acid molecule of the invention to be introduced into the one or more cells provided may include a regulatory sequence, preferably a promoter sequence, and optionally also a transcriptional termination sequence. The promoters may allow for either a constitutive or an inducible gene expression. Suitable promoters include *inter alia* the *E*. *coli lac*UV5 and *tet* (tetracycline-responsive) promoters, the T7 promoter as well as the SV40 promoter or the CMV promoter.

The nucleic acid molecules of the invention may also be comprised in a vector or other cloning vehicles, such as plasmids, phagemids, phages, cosmids or artificial chromosomes. In a preferred embodiment, the nucleic acid molecule is comprised in a vector, particularly in an expression vector. Such an expression vector can include, aside from the regulatory sequences described above and a nucleic acid sequence encoding a genetic construct as defined in the invention, replication and control sequences derived from a species compatible with the host that is used for expression as well as selection markers conferring a selectable phenotype on transfected cells. Large numbers of suitable vectors such as pSUPER and pSUPERIOR are known in the art, and are commercially available.

In a forth aspect, the invention relates to a pharmaceutical composition for the prevention and/or treatment in one or more mammalian target cells having a phenotype associated with a nasopharyngeal carcinoma, the composition comprising one or more nucleic acid molecules, each nucleic acid molecule encoding a sequence that is at least partially complementary to a microRNA sequence encoded by a nucleic acid molecule whose expression is up-regulated in the one or more target cells, as defined in the invention, and/or that corresponds to a microRNA sequence encoded by a nucleic acid molecule whose expression is down-regulated in the one or more target cells, as defined in the invention.

In preferred embodiments, the human nasopharyngeal carcinoma is associated with an infection caused by Epstein-Barr virus.

In a final aspect, the invention is directed to the use of such a pharmaceutical composition for the manufacture of a medicament for the prevention and/or treatment of a nasopharyngeal carcinoma, preferably a nasopharyngeal carcinoma associated with an infection caused by Epstein-Barr virus.

The ("sense" and "anti-sense") nucleic acid molecules described above can be formulated into compositions using pharmaceutically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA). To prepare the pharmaceutical compositions, pharmaceutically inert inorganic or organic excipients can be used. To prepare e.g. pills, tablets, capsules or granules, for example, lactose, talc, stearic acid and its salts, fats, waxes, solid or liquid polyols, natural and hardened oils may be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils.

The pharmaceutical composition may also contain additives, such as, for example, fillers, binders, wetting agents, glidants, stabilizers, preservatives, emulsifiers, and furthermore solvents or solubilizers or agents for achieving a depot effect. The latter is to be understood that the nucleic acid molecules may be incorporated into slow or sustained release or targeted delivery systems, such as liposomes, nanoparticles, and microcapsules.

To target most tissues within the body, clinically feasible noninvasive strategies are required for directing such pharmaceutical compositions, as defined herein, into cells. In the past years, several approaches have achieved impressive therapeutic benefit following intravenous injection into mice and primates using reasonable doses of siRNAs without apparent limiting toxicities.

One approach involves covalently coupling the passenger strand (miRNA* strand) of the miRNA to cholesterol or derivatives/conjugates thereof to facilitate uptake through ubiquitously expressed cell-surface LDL receptors (Soutschek, J. et al. (2004) Nature 432, 173-178). Another strategy for delivering miRNAs involves encapsulating the miRNAs into specialized liposomes formed using polyethylene glycol to reduce uptake by scavenger cells and enhance time spent in the circulation. These specialized nucleic acid particles (stable nucleic acid-lipid particles or SNALPs) delivered miRNAs effectively to the liver (and not to other organs (cf., e.g., Zimmermann, T.S. et al. (2006) Nature 441, 111-114). A further cell-specific targeting strategy involves the mixing of miRNAs with a fusion protein composed of a targeting antibody fragment linked to protamine, the basic protein that nucleates DNA in sperm and binds miRNAs by charge (Song, E. et al. (2005) Nat. Biotechnol. 23, 709-717). Multiple modifications or variations of the above basic delivery approaches have recently been developed. These techniques are known in the art and reviewed, e.g., in Kim, D.H. and Rossi, J.J. (2007) Nat. Genet. 8, 173-184; Elmen, J. et al. (2008) Nature 452, 896-899).

The invention is further described by the figures and the following examples, which are solely for the purpose of illustrating specific embodiments of this invention, and are not to be construed as limiting the scope of the invention in any way.

### EXAMPLES

### Example 1: Materials and Methods

### 1.1 Small RNA cloning

The small RNA fraction from tissue samples (each a NPC tumor and a healthy control tissue sample from a female and a male individual, respectively) was isolated using a mirVana-microRNA isolation kit (Ambion) and separated on a denaturing 12.5% polyacrylamide (PAA) gel stained with SYBRgreenII. After passive elution, RNAs with a length of 15-30 bases were concentrated by ethanol precipitation and dissolved in water. Next, RNAs were poly(A)-tailed using poly(A) polymerase, and an adapter was ligated to the 5'phosphate of the miRNAs: (5'end adapter (43 nucleotides): 5'-GCCTCCCTCGCGCCATCAGCTNNNNGACCTTGGCTGTCACTCA-3'). NNNN represents a "barcode" sequence for the individual samples: CAAT for NPC-1 (female), ATCG for CONTROL-1 (female), ACTA for NPC-2 (male), and AGGT for CONTROL-2 (male), respectively. Then, first strand cDNA synthesis was performed using an oligo(dT)-linker primer and M-MLV-RNase H⁻ reverse transcriptase (3'end oligo (dT) linker primer (61 bases): 5'-GCCTTGCCAGCCCGCTCAGACGAGACATCGCCCCGC(T)₂₅-3'). The cDNAs synthesized were PCR-amplified in 22 cycles using the high fidelity Phusion polymerase (Finnzymes). 120 to 135 bp amplification products were confirmed by polyacrylamide gel electrophoresis (PAGE) analysis. Both cDNAs pools were mixed in equal amounts and subjected to gel fractionation. The 120-135 bp fraction was electro-eluted from 6% PAA-gels. After isolation with Nucleospin Extract II (Macherey and Nagel) cDNA pools were dissolved in 5 mM Tris/HCl, pH 8.5 in a concentration of 10 ng/µl and used in single-molecule sequencing. Massively parallel sequencing was performed by 454 Life Sciences (Branford, USA) using the Genome Sequencer 20 system.

### 1.2 Sequence analysis

Base calling and quality trimming of sequence chromatograms were done by using the publicly available *Phred* software (Ewing, B et al. (1998) Genome Res. 8, 175-194). The adapter sequences and poly(A) tails were removed from the sequences. The remaining sequences having a length of 15 bases or more were subjected to later analysis. Due to the disturbing signal from poly(A) tails in 454 sequencing, the sequences were analyzed semi-manually, distinguishing the false "A" signals in the sequences.

The set of sequences were compared with the mature sequences of known miRNAs from miRBase (Griffiths-Jones, S. (2008) *supra*). The sequences that are not perfectly matching may be artifacts of the cloning procedure or a result of non-templated modification of mature miRNAs (Aravin, A. and Tuschl T. (2005) FEBS Lett. 579, 5830-5840) and were annotated according to the best blast hit to the database. The rest of the sequences were again compared with the hairpin sequences of known miRNAs from miRBase, and the star sequences of known miRNAs that have not been annotated in the database were identified. Subsequently, the sequences were subject to the blast against non-coding RNA sequences, including rRNA, tRNA, snoRNA etc., retrieved from Genbank (http://www.ncbi.nlm.nih.gov/Genbank) and the annotated sequences were discarded from the sequence set. The non-coding RNA sequences from EBV were identified by the blast against EBV genome retrieved from Genbank.

The rest of sequences, including the un-annotated sequences from EBV and human genomes, were then subjected to the search for putative novel miRNA sequences. Genomic regions containing the inserts with 100 nt flanks were retrieved from ENSEMBL to calculate RNA secondary structures by MFOLD (Zuker, M. (2003) Nucleic Acids Res. 31, 3406-3415). Only regions that folded into hairpins and contained an insert in one of the hairpin arms were considered as putative novel miRNA sequences.

### 1.3. Cell Culture

BL41, BL41/B95.8, and Jijoye cells were grown in RPMI 1640 medium with L-glutamine, supplemented with 10% heat-inactivated fetal bovine serum (FBS), 100 unit/ml penicillin, and 100 µg/ml streptomycin at 37°C in atmosphere containing 5% CO₂.

### 1.4. Northern Blotting

Total RNA was isolated from BL41, BL41/B95.8, and Jijoye cells by using Trizol reagent (Invitrogen) according to the manufacturer's instructions, and separated by 15% denaturing RNA PAGE. Northern Blotting against miRNAs was performed as described previously (Lagos-Quintana, M. et al. (2001) Science 294, 853-858). The probe sequences used for detecting new miRNA candidates were: ebv-mir-BART-21-5P, 5'-GTTAGTTGCCTT CACT AGTGA-3'; ebv-mir-BART-21-3P, 5'-AAACACCAGTGGGCACAACTAG-3'; ebv-mir-BART-22, 5'-ACTACTAGACCATGACTTTGTA-3'; and ebv-mir-BART-4-3P, 5'-ACACCTGGTGCCTACGTGAT GTG-3'.

### Example2: Small RNA libraries from NPC and control tissues

In order to investigate a possible contribution of cellular or EBV-derived miRNAs to NPC pathology, small RNA profiles from different EBV-positive NPC tissues were established. Total RNA from two NPC samples (NPC 1 and NPC2) and corresponding control tissues (CONTROL1 and CONTROL2) obtained from the throat of the same patients was extracted and small RNAs were cloned according to an established cloning protocol.

The individual libraries were further analyzed by pyro-sequencing using 454 technologies. 25.223 sequence reads for NPC-1 and 22.636 reads for NPC-2 were obtained that were grouped due to their origin (cf. Table 1). For control tissues, 16.249 (CONTROL-1) and 19.052 (CONTROL-2) sequences were analyzed. Interestingly, only for the NPC tissues more than 50% of the sequences could be mapped to the human or the EBV genome. In contrast, the libraries obtained from the control tissues contained only a minor portion of human or EBV-derived sequences. The majority of the sequences were derived from bacterial genomes most likely due to the bacterial flora in the throat of the patients.

About 20.000 sequences of NPC-1 and about 6.000 sequences of NPC-2 were identified as known cellular or viral miRNAs. In the control tissues, significantly lower numbers of miRNAs were found, presumably due to the presence of high numbers of bacterial RNAs within the libraries. Notably, the numbers of sequences from other non-coding RNAs are about 10% for NPC-1, CONTROL-1, and CONTROL-2. However, the library from NPC-2 contained about 40 % other non-coding RNAs indicative for RNA degradation and therefore lower RNA quality (cf. Table1).

### Example 3: Identification of new viral and cellular miRNAs

So far, many miRNA genes have been identified in various organisms. However, bioinformatic predictions suggest that many more miRNA genes may exist at least in mammalian genomes. Therefore, the data sets otained were analyzed for novel miRNA candidates. Initially, sequences were investigated that match to the EBV genome but have not been functionally annotated yet. Unexpectedly, three different small RNAs deriving form precursors that fold to hairpins were found (cf. Table 2, Figure 1A). Two of the three RNAs are encoded by the 5' and the 3' arm of the same hairpin. These miRNAs were designated ebv-miR-BART21-5P, ebv-miR-BART21-3P, and ebv-miR-BART22. Interestingly, ebv-miR-BART21 is conserved in *Rhesus* lymphocryptovirus (rLCV), a common model system for EBV infection (cf. Table 2).

The expression levels of the novel EBV-miRNA candidates were analyzed by Northern blotting (cf. Figure 1B). Total RNA from the EBV-positive cell lines Jijoye and B141/B95.8 as well as the EBV-negative B-cell line BL-41 was separated by RNA PAGE and transferred to a nylon membrane. Probes specific to the two arms of the miR-BART21 candidate as well as miR-BART22 were readily detected in Jijoye cells, whereas no signal was observed in BL41B95.8 and BL41 cells. Notably, the BL41-B95.8 cell line carries a deletion within the BART region where the two novel miRNAs are located. Furthermore, star (*) sequences for ebv-miR-BART4 and ebv-miR-BART5 were detected in the NPC samples suggesting that viral miRNA* molecules might be functional as well (cf. Table 3).

In order to find novel human miRNAs, the pool of unknown sequences was analyzed that match to the human genome. Indeed, three novel hsa-miRNA genes were identified. According to miRBase these miRNAs were designated hsa-miR-945, hsa-miR-946 and hsa-miR-947 (cf. Table 2). hsa-miR-945 is located in an intergenic region, whereas hsa-miR-946 and hsa-miR-947 reside in the 3'UTRs of protein coding genes (cf. Table 2).

In summary, three novel EBV miRNAs as well as three novel human miRNA genes were identified. Additionally, six so far unidentified miRNA* sequences were found that derive from known miRNA genes and have not been cloned before (cf. Table 3).

### Example 4: miRNA profile from NPC tissue

MicroRNA expression analyses revealed distinct miRNA signatures for different types of tumors (Calin, G.A., and Croce, C.M. (2006) Nat. Rev. Cancer 6, 857-866). In order to establish a miRNA signature characteristic for NPCs, the most abundant miRNAs in the libraries were identified (cf. Figure 2 and Table 4).

About 5% of the NPC-1 and 19% of the NPC-2 miRNAs were classified as EBV miRNAs indicating that viral miRNAs are highly expressed in NPCs (cf. Figure 2A). Among the EBV miRNAs high levels of ebv-miR-BART1, ebv-miR-BART4, ebv-miR-BART6, ebv-miR-BART7, ebv-miR-BART11, ebv-miR-BART12, ebv-miR-BART19 as well as the new miRNAs ebv-miR-BART21-5p, ebv-miR-BART21-3p, and ebv-miR-BART22 were found. Interestingly, no EBV miRNA from the BHRF1 region was identified in our libraries suggesting that miRNAs from this region might not be involved in NPC biology (cf. Figure 2B).

The most abundant human miRNAs are presented in Figure 2C. The twenty most frequently cloned miRNAs are similar between NPC and control tissues (cf. Table 4). However, hsa-miR-320, hsa-miR-17-5p, and hsa-miR-652 are less present in the libraries obtained from the NPCs compared to those obtained from the control tissues suggesting that these miRNAs might be down regulated in NPC tissue. Furthermore, the numbers for hsa-miR-23a/b (both miRNAs were frequently indistinguishable in the sequencing data) and hsa-miR-200c were significantly increased in the NPC tumor samples suggesting up-regulation of these miRNAs.

Using small RNA cloning and sequencing the miRNA expression profile of EBV-positive NPC tissues was characterized. It was found that EBV expresses all miRNAs from the BART family in NPC tissues suggesting that these miRNAs may have an important function for maintaining the virus in NPC tissues. Moreover, two novel EBV miRNAs were identified, namely ebv-miR-BART-21 and ebv-miR-BART-22. Notably, no miRNA originating form the BHRF region of the EBV genome was found in the datasets suggesting, that these miRNAs might not be important for NPC pathology.

Like other tumors, NPC samples are characterized by distinct target cell miRNA expression profiles. In particular, the results demonstrate that hsa-miR-23a/b and hsa-miR-200c are significantly up-regulated, whereas hsa-miR-320, hsa-miR-17-5p, and hsa-miR-652 are down-regulated in NPC samples. Therefore, the data might be valuable for identifying and classifying NPCs probably even at very early stages. Moreover, interfering with over-represented miRNAs or artificial expression of under-represented miRNAs might open new ways for NPC therapy.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

**Table 1: Small RNA read numbers obtained from NPC libraries and controls.**

| | NPC-1 | CONTROL-1 | NPC-2 | CONTROL-2 |
|---|---|---|---|---|
| **Total # of reads** | **25223** | **16249** | **22636** | **19052** |
| # Mapped to human genome | 21668 | 1874 | 13445 | 3330 |
| # Mapped to EBV genome | 1014 | 34 | 1217 | 0 |
| **# Known miRNAs** | **20026** | **1269** | **5980** | **1445** |
| # Human miRNAs | 19261 | 1242 | 4893 | 1445 |
| # EBV miRNAS | 758 | 27 | 1081 | 0 |
| **# Human ncRNAs** | **2317** | **616** | **8412** | **1843** |
| # rRNAs | 1294 | 462 | 7185 | 1456 |
| # tRNAs | 156 | 90 | 392 | 117 |
| # other ncRNAs | 867 | 64 | 835 | 270 |

**Table 2: Novel microRNA genes identified in NPC samples**

| **Candidate miRNA¹** | **Stem-loop structures (SLS) of putative miRNA precursors²** | **dG²** | **Genomic location** | **Conservation of SLS** |
|---|---|---|---|---|
| **EBV miRNA** | | | | |
| *ebv-mir-BART21-5P* | UU AAG A AGU GGGCUGGGUA CACUAGUG GCAACUA CAC U CCUGGCCUAU GUGGUCAC UGUUGAU GUG A UU CCG C CAG | -40.4 | 145514-145534³ | rLCV⁵ |
| *ebv-mir-BART21-3P* | | -40.4 | 145548-145569³ | rLCV⁵ |
| *ebv-mir-BART22* | G                    C   AG                    UA GUCACAG  UGCUAGACC  UGG    UUG-AACC- AG     \ CAGUGUU  AUGAUCUGG ACU    AAC  UUGG  UC     C G                    U    GA      A        C   AC | - 35.8 | 147203-147225³ | |

| **Human miRNA** | | | | |
|---|---|---|---|---|
| *hsa-mir-945* |           G                              CC                GUG GGGGAUUGUG  GGGGUCGCU - CUAGGCA   GCAGCA- CU        \ CUCCUGACAC  CUUCAGUGAGGGUCCGU   CGUUGUAGG        C   · A                              CGA               GGU | - 47.2 | 2p23.3⁴ | mmu⁵ |
| *hsa-mir-946* | CCA U A- U C UC UG ACUGCCUA AUC CGACCGG CC CGACCGG UCG UG C UGGCGGAU UGG GCUGGCU GG GC- GGCU AGC AC U AGA U GC U C UA CG | - 39.6 | 10q24.33⁴ | ptr, mml⁵ |
| *hsa-mir-947* |         UU              C                    U    G      UC CAGGGGU   GGGGAAA GGCCGC - - UGAG  GA  GCG    \ GUUCUCA    CUCCUUU  CUGGCG    ACUC  UU  UGU    G CC              U              CC       U     G      C | - 34.1 | 10q25.3⁴ | |

| | | | | |
|---|---|---|---|---|
| 1 The names of the putative new miRNA sequences are according to the submission to miRBase (http://microrna.sanger.ac.uk/sequences). 2 RNA secondary structure predictions and free energy calculations were done using MFOLD version 3.2. The miRNA sequences are underlined. 3 The genomic location of EBV miRNAs are referring to the positions in EBV genome sequence of accession number AJ507799 from GenBank (http://www.ncbi.nlm.nih.gov/Genbank) 4 The genomic location of human miRNAs are referring to the positions in human chromosomes according to the assembly from Ensembl (http://www.ensembl.org). 5 rLCV, Rhesus lymphocryptovirus; mmu, *Mus musculus* (mouse); ptr, *Pan troglodytes* (chimpanzee); mml, *Macaca mulatta* (monkey). | | | | |

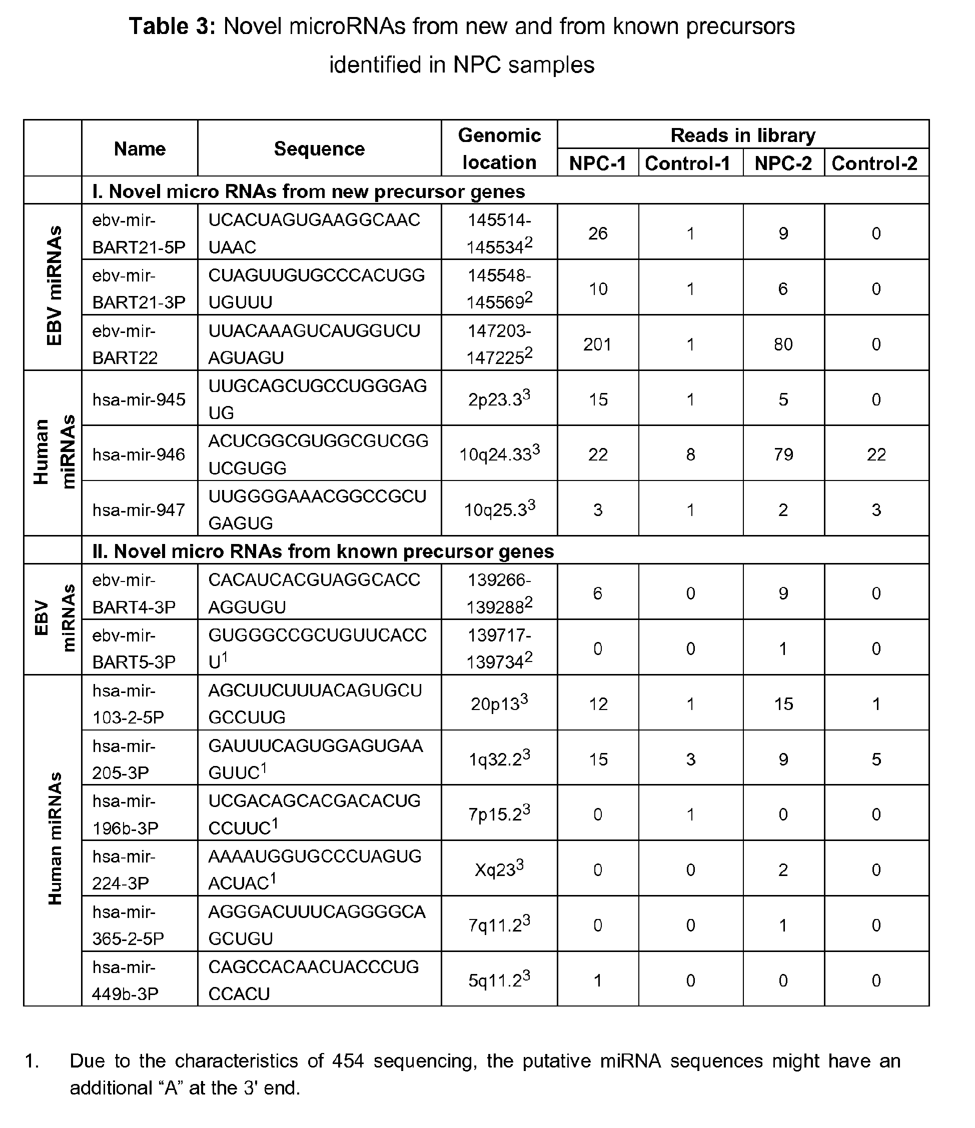

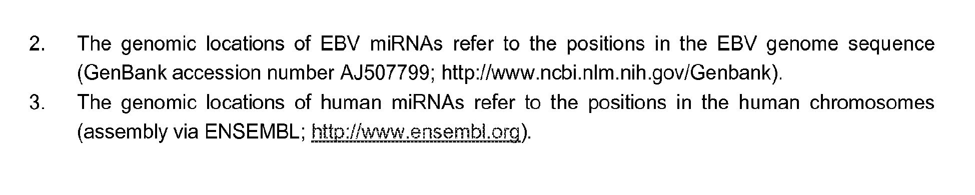

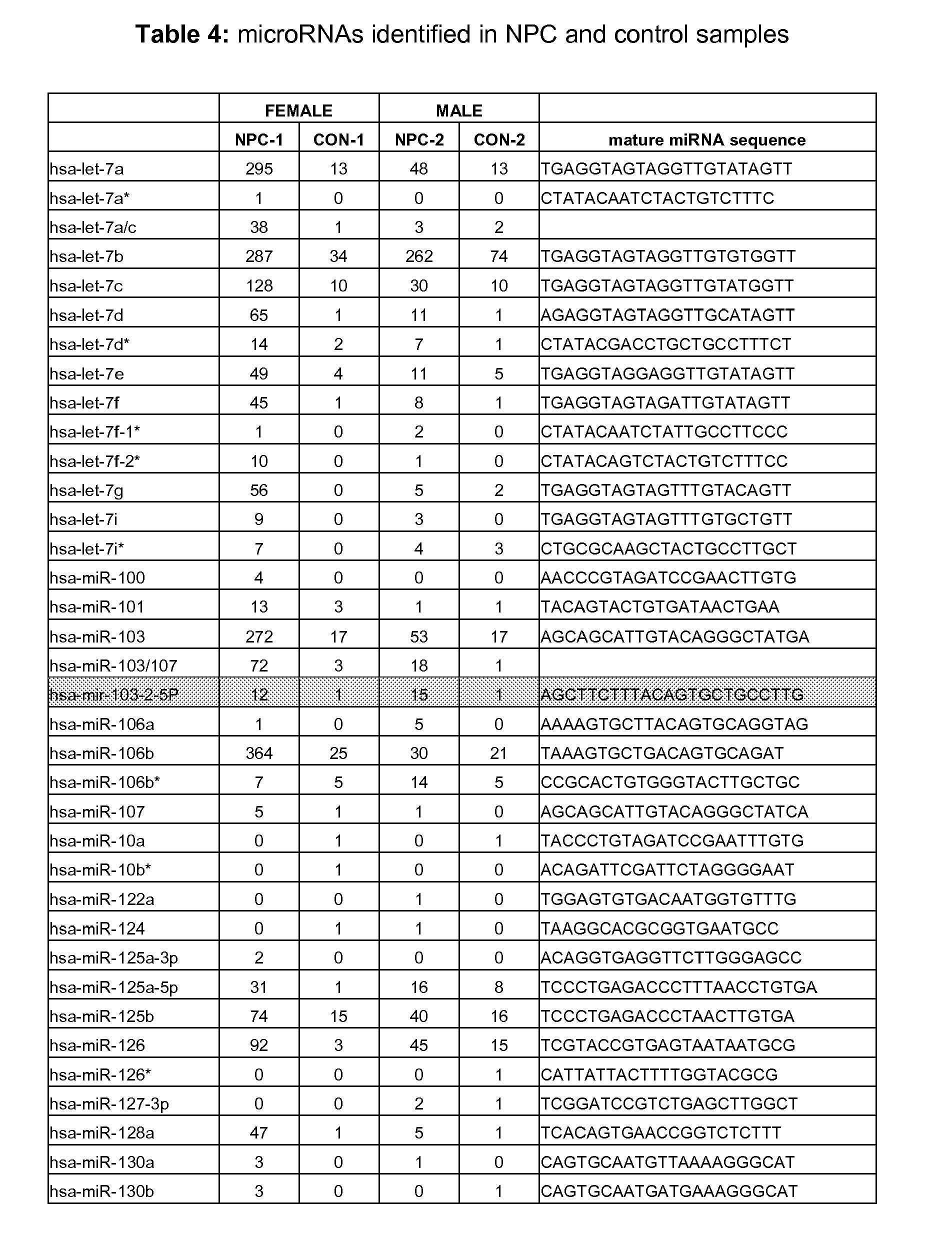

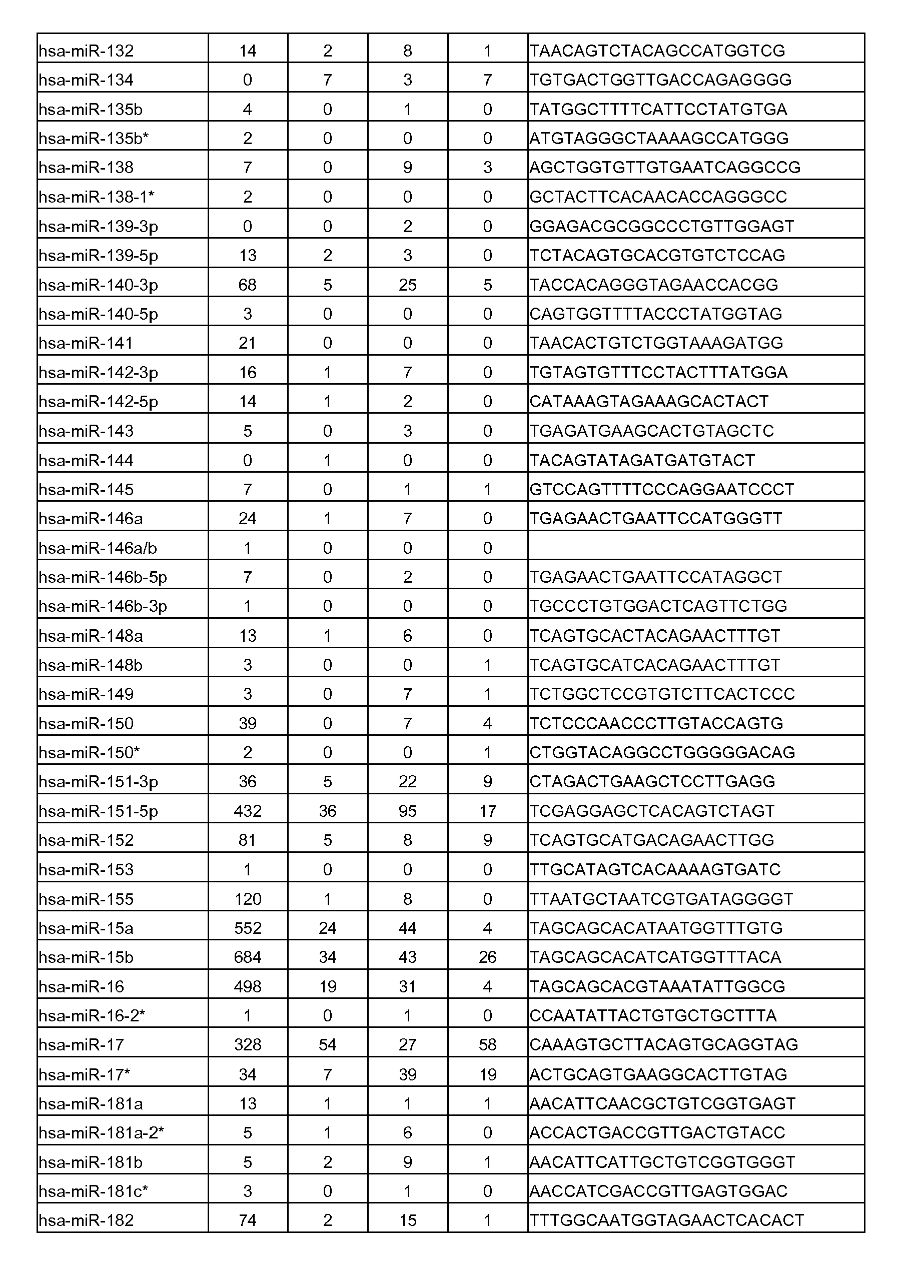

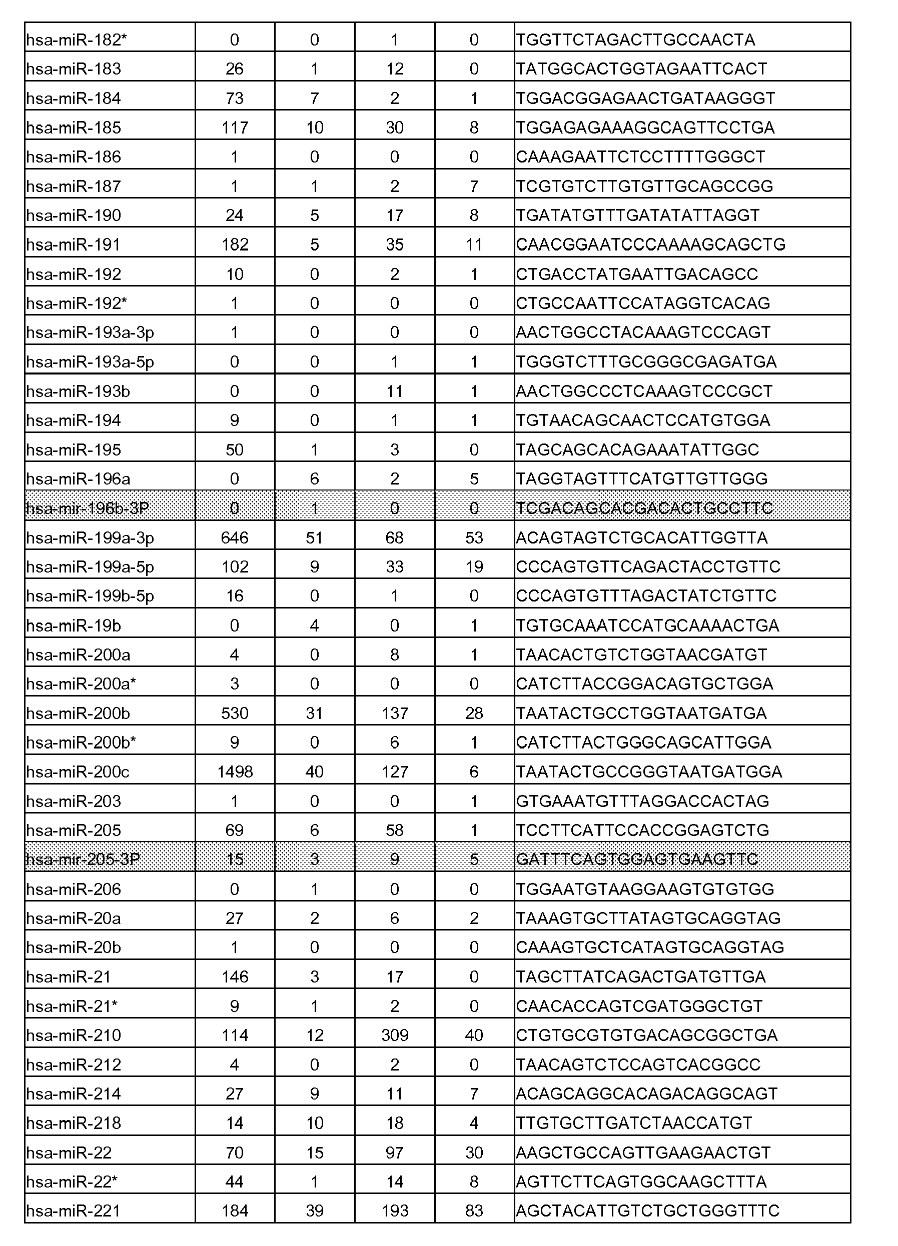

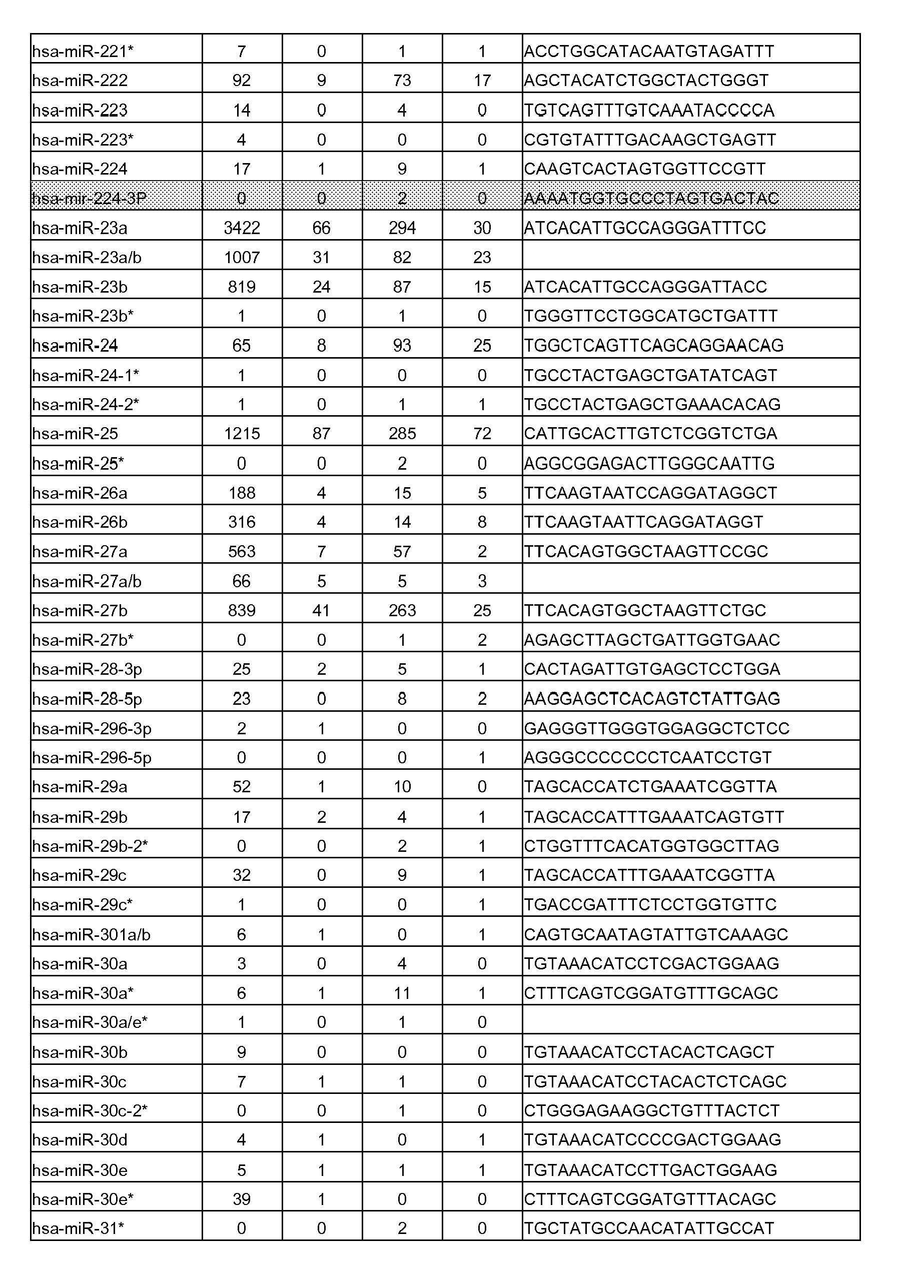

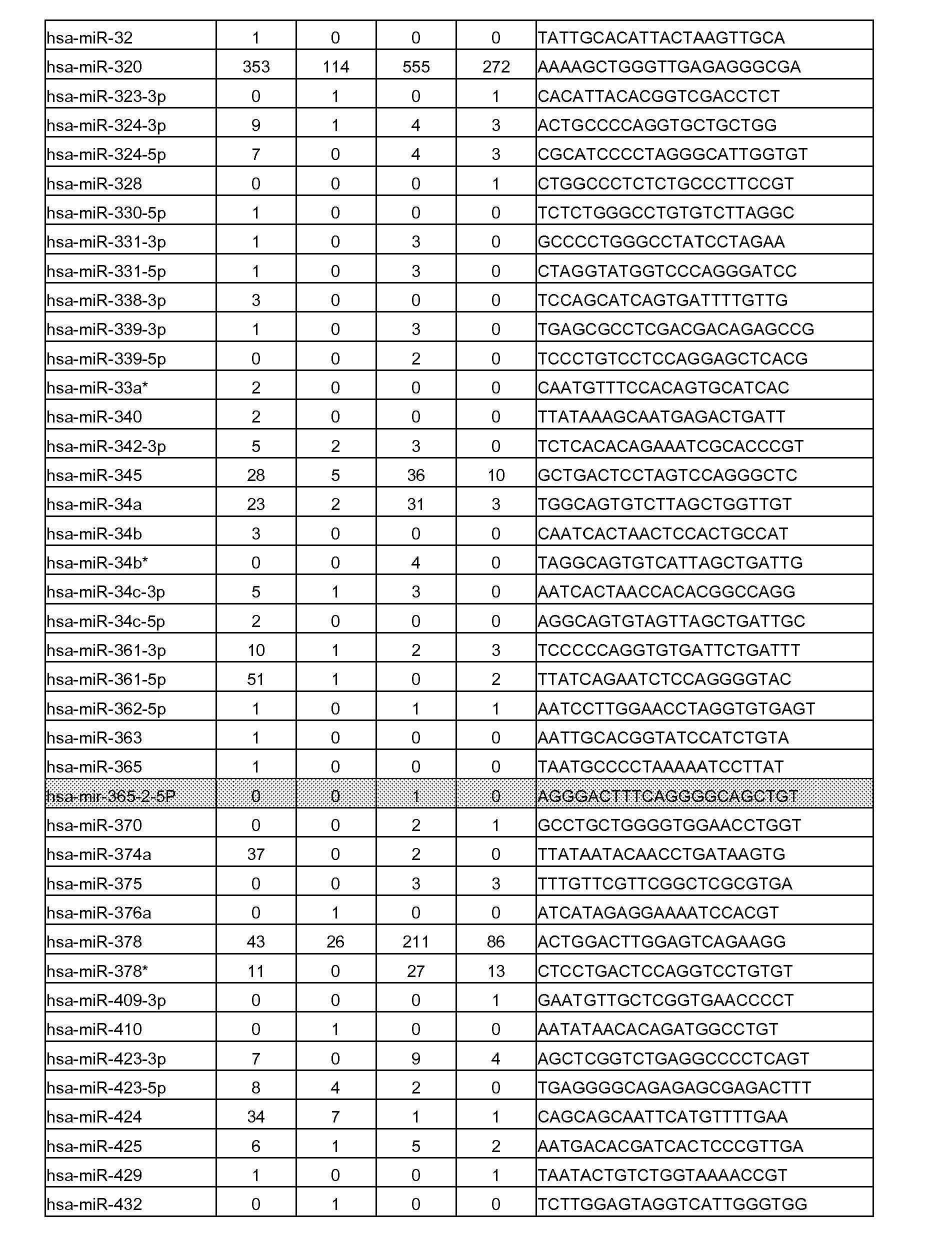

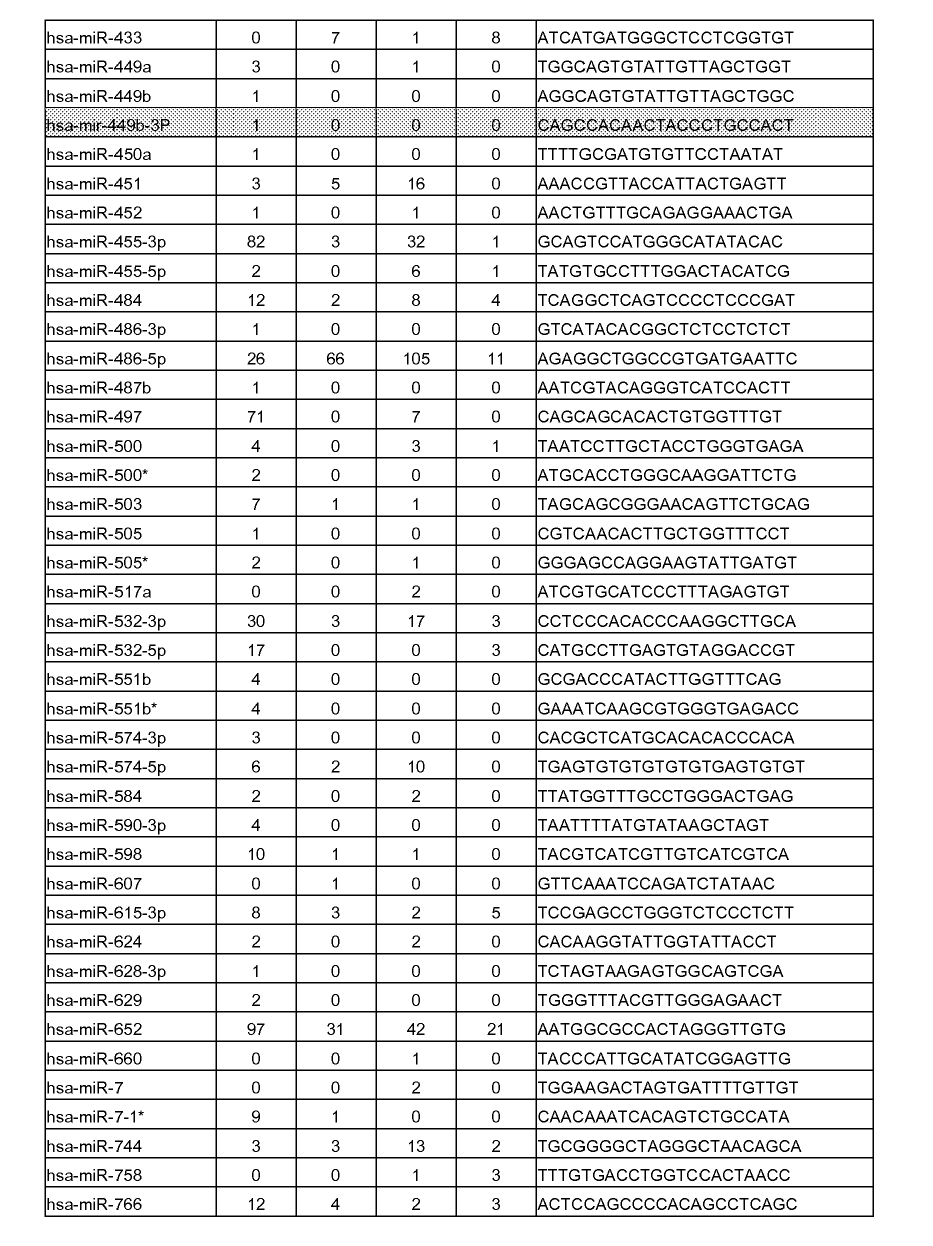

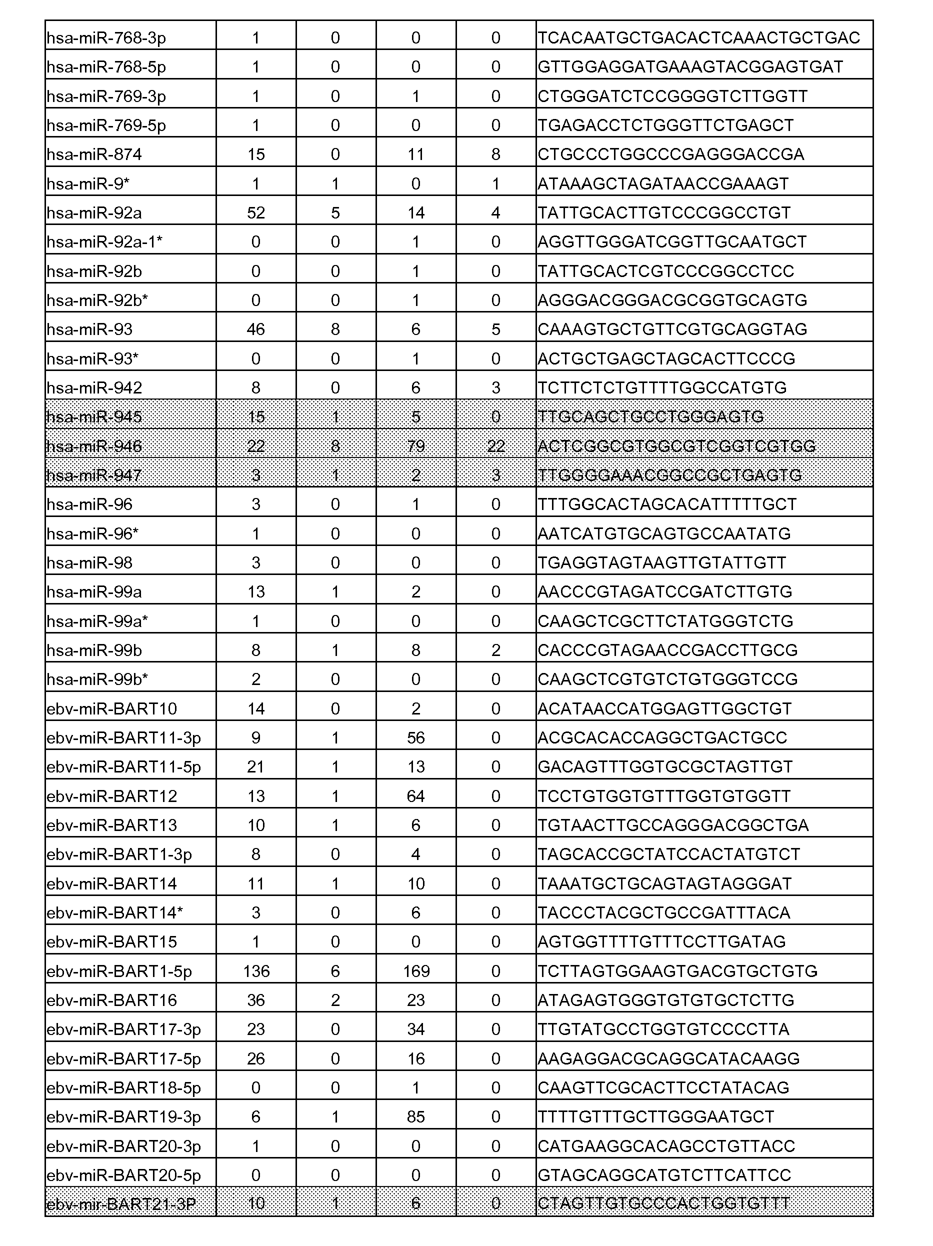

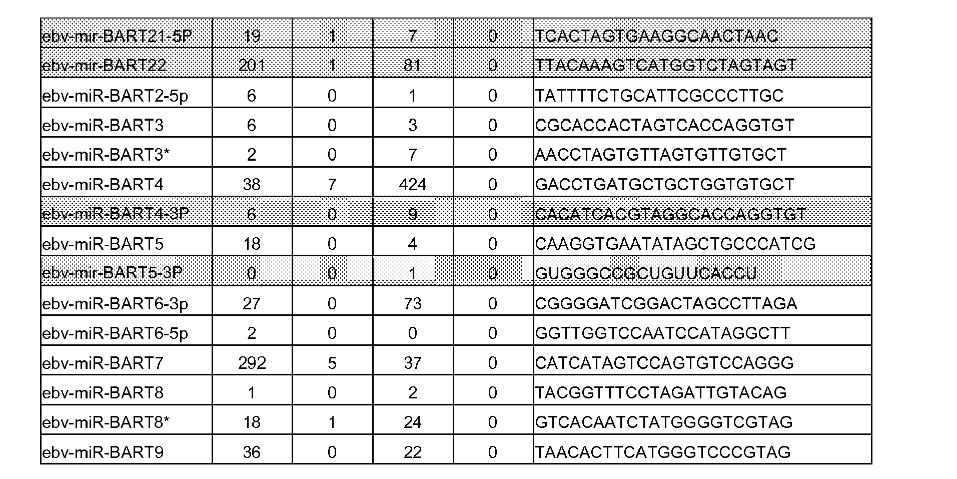

The microRNAs shown in grey boxes are the novel microRNAs identified. "/" is used whenever two microRNAs cannot be distinguished because of only subtle differences.

## Claims

1. Diagnostic kit of molecular markers for identifying one or more mammalian target cells exhibiting or having a predisposition to develop a phenotype associated with a nasopharyngeal carcinoma, the kit comprising a plurality of nucleic acid molecules, each nucleic acid molecule encoding a microRNA sequence, wherein one or more of the plurality of nucleic acid molecules are differentially expressed in the target cells and in one or more healthy control cells, and wherein the one or more differentially expressed nucleic acid molecules together represent a nucleic acid expression signature that is indicative for the presence of or the predisposition to develop a phenotype associated with a nasopharyngeal carcinoma.

2. The kit of claim 1, wherein the nasopharyngeal carcinoma is associated with a viral infection, preferably a viral infection caused by Epstein-Barr virus.

3. The kit of claim 1 or 2, wherein the nucleic acid expression signature comprises at least five nucleic acid molecules, preferably at least ten nucleic acid molecules.

4. The kit of claim 2 or 3, wherein the nucleic acid expression signature comprises at least one nucleic acid molecule encoding a target cell-derived microRNA sequence and at least one nucleic acid molecule encoding a virus-derived microRNA sequence.

5. The kit of any of claims 2 to 4, wherein the nucleic acid expression signature comprises at least one nucleic acid molecule encoding a microRNA sequence whose expression is up-regulated in the one or more target cells compared to the one or more control cells and at least one nucleic acid molecule encoding a microRNA sequence whose expression is down-regulated in the one or more target cells compared to the one or more control cells.

6. The kit of any of claims 6 to 5, wherein the nucleic acid expression signature comprises one or more human target cell-derived nucleic acid molecules encoding microRNA sequences selected from the group consisting of hsa-miR-23a, hsa-miR-23b, hsa-miR200c, hsa-miR-320, hsa-miR-17-5p, and hsa-miR-652.

7. The kit of any of claims 2 to 6, wherein the nucleic acid expression signature comprises one or more Epstein-Barr virus-derived nucleic acid molecules encoding microRNA sequences selected from the group consisting of ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22.

8. The kit of any of claims 2 to 7, wherein the nucleic acid expression signature comprises nucleic acid molecules encoding hsa-miR-23a, hsa-miR-23b, hsa-miR200c, hsa-miR-320, hsa-miR-17-5p, hsa-miR-652, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22.

9. The kit of claim 8, wherein the expression of the nucleic acid molecules encoding hsa-miR-23a, hsa-miR-23b, hsa-miR200c, ebv-miR-BART1-5p, ebv-miR-BART4, ebv-miR-BART6-3p, ebv-miR-BART7-3p, ebv-miR-BART11-3p, ebv-miR-BART12, ebv-miR-BART19-3p, and ebv-miR-BART22 is up-regulated and the expression of the nucleic acid molecules hsa-miR-320, hsa-miR-17-5p, and hsa-miR-652 is down-regulated in the in the one or more target cells compared to the one or more control cells.

10. Method for identifying one or more mammalian target cells exhibiting or having a predisposition to develop a phenotype associated with a nasopharyngeal carcinoma, the method comprising:
(a) determining in the one or more target cells the expression levels of a plurality of nucleic acid molecules, each nucleic acid molecule encoding a microRNA sequence;
(b) determining the expression levels of the plurality of nucleic acid molecules in one or more healthy control cells; and
(c) identifying from the plurality of nucleic acid molecules one or more nucleic acid molecules that are differentially expressed in the target and control cells by comparing the respective expression levels obtained in steps (a) and (b),
wherein the one or more differentially expressed nucleic acid molecules together represent a nucleic acid expression signature, as defined in any of claims 3 to 9, that is indicative for the presence of or the predisposition to develop a phenotype associated with a nasopharyngeal carcinoma.

11. The method of claim 10, wherein the nasopharyngeal carcinoma is associated with a viral infection, preferably a viral infection caused by Epstein-Barr virus.

12. Method for preventing or treating a phenotype associated with a nasopharyngeal carcinoma in one or more mammalian target cells, the method comprising:
(a) identifying in one or more target cells a nucleic acid expression signature by using a method as defined in claim 10 or 11; and
(b) modifying in the one or more cells the expression of one or more nucleic acid molecules encoding a microRNA sequence that is/are comprised in the nucleic acid expression signature in such way that the expression of a nucleic acid molecule whose expression is up-regulated in the one or more target cells is down-regulated and the expression of a nucleic acid molecule whose expression is down-regulated in the one or more target cells is up-regulated.

13. Pharmaceutical composition for the prevention and/or treatment in one or more mammalian target cells having a phenotype associated with a nasopharyngeal carcinoma, the composition comprising one or more nucleic acid molecules, each nucleic acid molecule encoding a sequence that is at least partially complementary to a microRNA sequence encoded by a nucleic acid molecule whose expression is up-regulated in the one or more target cells, as defined in any of claims 3 to 9, and/or that corresponds to a microRNA sequence encoded by a nucleic acid molecule whose expression is down-regulated in the one or more target cells, as defined in any of claims 3 to 9.

14. The pharmaceutical composition of claim 13, wherein the human nasopharyngeal carcinoma is associated with an infection caused by Epstein-Barr virus.

15. Use of the pharmaceutical composition of claim 13 or 14 for the manufacture of a medicament for the prevention and/or treatment of a nasopharyngeal carcinoma, preferably a nasopharyngeal carcinoma associated with an infection caused by Epstein-Barr virus.


SEQUENCE LISTING

<110> Max-Planck-Gesellschaft zur Förderung der Wissenschaften e.V.
      Universitätsklinikum Homburg
<120> Compositions and methods for micro-RNA expression profiling of
      nasopharyngeal carcinoma

<130> M 9548 / DB

<140> not assigned yet
<141> 2008-04-24

<160> 28

<170> PatentIn Version 3.4


<210> 1
<211> 21
<212> RNA
<213> Homo sapiens
      hsa-miR-23a microRNA

<400> 1

aucacauugc cagggauuuc c                                                21


<210> 2
<211> 74
<212> RNA
<213> Homo sapiens
      hsa-miR-23a microRNA precursor

<400> 2

ggccggcugg gguuccuggg gaugggauuu gcuuccuguc acaaaucaca uugccaggga      60
uuuccaaccg acc                                                         73


<210> 3
<211> 21
<212> RNA
<213> Homo sapiens
      hsa-miR-23b microRNA

<400> 3

aucacauugc cagggauuac c                                                21


<210> 4
<211> 97
<212> RNA
<213> Homo sapiens
      hsa-miR-23b microRNA precursor

<400> 4

cucaggugcu cuggcugcuu ggguuccugg caugcugauu ugugacuuaa gauuaaaauc 60
acauugccag ggauuaccac gcaaccacga ccuuggc 97

<210> 5
<211> 23
<212> RNA
<213> Homo sapiens
      hsa-miR-200c microRNA

<400> 5

uaauacugcc ggguaaugau gga 23

<210> 6
<211> 68
<212> RNA
<213> Homo sapiens
      hsa-miR-200c microRNA precursor

<400> 6

cccucgucuu acccagcagu guuugggugc gguugggagu cucuaauacu gccggguaau 60
gauggagg 68

<210> 7
<211> 22
<212> RNA
<213> Homo sapiens
      hsa-miR-320 microRNA

<400> 7

aaaagcuggg uugagagggc ga 22

<210> 8
<211> 82
<212> RNA
<213> Homo sapiens
      hsa-miR-320 microRNA precursor

<400> 8

gcuucgcucc ccuccgccuu cucuucccgg uucuucccgg agucgggaaa agcuggguug 60
agagggcgaa aaaggaugag gu 82

<210> 9
<211> 23
<212> RNA
<213> Homo sapiens
      hsa-miR-17-5p microRNA

<400> 9

caaagugcuu acagugcagg uag 23

<210> 10
<211> 84
<212> RNA
<213> Homo sapiens
      hsa-miR-17-5p microRNA precursor

<400> 10

gucagaauaa ugucaaagug cuuacagugc agguagugau augugcaucu acugcaguga      60
aggcacuugu agcauuaugg ugac                                             84


<210> 11
<211> 21
<212> RNA
<213> Homo sapiens
      hsa-miR-652 microRNA

<400> 11

aauggcgcca cuaggguugu g                                                21


<210> 12
<211> 98
<212> RNA
<213> Homo sapiens
      hsa-miR-652 microRNA precursor

<400> 12

acgaauggcu augcacugca caacccuagg agagggugcc auucacauag acuauaauug      60
aauggcgcca cuaggguugu gcagtgcaca accuacac                              98


<210> 13
<211> 24
<212> RNA
<213> Epstein Barr virus
      ebv-miR-BART1-5p microRNA

<400> 13

ucuuagugga agugacgugc ugug                                             24


<210> 14
<211> 70
<212> RNA
<213> Epstein Barr virus
      ebv-miR-BART1-5p microRNA precursor

<400> 14

gggggucuua guggaaguga cgugcuguga auacaggucc auagcaccgc uauccacuau      60
gucucgcccg                                                             70

<210> 15
<211> 22
<212> RNA
<213> Epstein Barr virus
      ebv-miR-BART4 microRNA

<400> 15

gaccugaugc ugcuggugug cu                                                22


<210> 16
<211> 76
<212> RNA
<213> Epstein Barr virus
      ebv-miR-BART4 microRNA precursor

<400> 16

uuugguggga ccugaugcug cuggugugcu guaaauaagu gccuagcaca ucacguaggc      60
accagguguc accagg                                                       76


<210> 17
<211> 22
<212> RNA
<213> Epstein Barr virus
      ebv-miR-BART6-3p microRNA

<400> 17

cggggaucgg acuagccuua ga                                                22


<210> 18
<211> 92
<212> RNA
<213> Epstein Barr virus
      ebv-miR-BART6-3p microRNA precursor

<400> 18

ugaccuuguu gguacuuuaa gguuggucca auccauaggc uuuuuuugug aaaacccggg      60
gaucggacua gccuuagagu aacucaaggc ca                                     92


<210> 19
<211> 22
<212> RNA
<213> Epstein Barr virus
      ebv-miR-BART7-3p microRNA

<400> 19

caucauaguc caguguccag gg                                                22


<210> 20
<211> 86

<212> RNA
<213> Epstein Barr virus
      ebv-miR-BART7-3p microRNA precursor

<400> 20

uccagugucc ugauccugga ccuugacuau gaaacaauuc uaaaaaaaug caucauaguc      60
caguguccag ggacagugca cucgga                                           86


<210> 21
<211> 21
<212> RNA
<213> Epstein Barr virus
      ebv-miR-BART11-3p microRNA

<400> 21

acgcacacca ggcugactgc c                                                21


<210> 22
<211> 86
<212> RNA
<213> Epstein Barr virus
      ebv-miR-BART11-3p microRNA precursor

<400> 22

ggcuucuguu gggucagaca guuuggugcg cuaguugugu gcuuagcagc aacgcacacc      60
aggcugacug ccuuagcagu guggcc                                           86


<210> 23
<211> 22
<212> RNA
<213> Epstein Barr virus
      ebv-miR-BART12 microRNA

<400> 23

uccuguggug uuuggugugg uu                                               22


<210> 24
<211> 83
<212> RNA
<213> Epstein Barr virus
      ebv-miR-BART12 microRNA precursor

<400> 24
   cuggugaccu aacacccgcc caucaccacc ggacagauuc ugaacuuguc cugugguguu      60
   uggugugguu uugggguacg cag                                               83


<210> 25
<211> 21
<212> RNA
<213> Epstein Barr virus

ebv-miR-BART19-3p microRNA

<400> 25

uuuuguuugc uugggaaugc u                                              21


<210> 26
<211> 83
<212> RNA
<213> Epstein Barr virus
      ebv-miR-BART19-3p microRNA precursor

<400> 26

guauccgugu ccugacaaca uuccccgcaa acaugacaug gguuaauuua aacauguuuu      60
guuugcuugg gaaugcucuu agggccugga agc                                   83


<210> 27
<211> 23
<212> RNA
<213> Epstein Barr virus
      ebv-miR-BART22 microRNA

<400> 27

uuacaaaguc auggucuagu agu                                              23


<210> 28
<211> 71
<212> RNA
<213> Epstein Barr virus
      ebv-miR-BART22 microRNA precursor

<400> 2

gucacaggug cuagacccug gaguugaacc aguaccacuc gguuacaaag ucauggucua      60
guaguuguga c                                                           71